# EUROPEAN PATENT SPECIFICATION

(11) **EP 4 225 812 B1**
(45) Date of publication and mention of the grant of the patent: **18.09.2024**
(21) Application number: 21799190.0
(22) Date of filing: 07.10.2021
(51) Int. Cl.: B33Y 70/00, B33Y 80/00, C07D 215/20, C07D 215/227, C08F 2/50, C08F 222/10, C09D 4/00, C09D 11/101, C07D 215/36

(54) **KETOQUINOLONES AS PHOTONITIATORS**
KETOCHINOLONE ALS PHOTONITIATOREN
CÉTOQUINOLONES UTILISÉES EN TANT QUE PHOTONITIATEURS

(30) Priority: 09.10.2020 IT 202000023815
(43) Date of publication of application: 16.08.2023
(73) Proprietor: IGM Resins Italia S.r.l., 20154 Milano (IT)
(72) Inventor: MORONE, Marika, 22030 Lipomo (CO) (IT); RAZZANO, Vincenzo, 20060 Bussero (MI) (IT); NORCINI, Gabriele Pietro, 21020 Comabbio (VA) (IT); POSTLE, Stephen, Bradenton, Florida 34211 (US)
(74) Representative: Gislon, Gabriele
(86) International application number: PCT/EP2021/077697
(87) International publication number: WO 2022/074120

(56) References cited:
- WO-A1-2014/063997
- WO-A1-92/17452
- MALINOWSKI ZBIGNIEW ET AL: "Synthesis of benzoquinoline derivatives from formyl naphthylamines via Friedländer annulation under metal-free conditions", MONATSHEFTE FÜR CHEMIE / CHEMICAL MONTHLY, SPRINGER VIENNA, VIENNA, vol. 149, no. 11, 15 September 2018 (2018-09-15), pages 1999 - 2011, XP036606043, ISSN: 0026-9247, [retrieved on 20180915], DOI: 10.1007/S00706-018-2268-X

## Description

### SUMMARY OF THE INVENTION

The present invention relates to novel class of photoinitiators and their use in photopolymerization compositions. The invention also concerns a process for the photopolymerization of compositions comprising said photoinitiators.

### PRIOR ART

The dynamic growth of UV curing is dependent on continued innovation to support this technology in the overcoming of always new challenges and this is reflected by rapid development of new materials needed for the formulations. In particular, one key component is the photoinitiator (PI), its role is that to convert light to chemical energy in the form of reactive intermediates. These intermediates are radicals able to initiate radical polymerization of the double bonds present in the formulation to be cured. The development in the field of photoinitiators is stimulated by various factors. Firstly, there is a continuous improvement of photoinitiators for existing applications such as coatings, inks, adhesives or electronics because there is no single photoinitiator that is able to meet all the requirements of each application, like for instance line speed, surface curing, no discoloration after curing or solubility. Secondly, the introduction of new lamps, such as LED lamps, stimulated the development of photoinitiators tuned at that wavelength. Thirdly, the increasing number of photoinitiators banned as toxic or reprotoxic. WO 2014/063997 A1, for example, describes photocurable compositions including ketocoumarine compounds as photoinitiators.

So, the research of new photoinitiators is always required in order to mimic standard photoinitiators or to overcome problems such as yellowing, or to higher line speed, curing under LED lamps, deep curing.

In recent years, some new classes were explored, some examples are acylgermanium photoinitiators (EP3150641, EP2649981), benzoyl phenyltelluride PIs (Macromolecules, 2014, 47(16), 5526-5531), silicon based PIs (JP2010229169, Macromolecules, 2009, 5 42(16), 6031-6037, Macromolecules 2007, 40(24), 8527-8530, Macromol. Rapid Commun. 2017, 38, 1600470, Macromolecules, 2017, 50(17), 6911-6923). Ketoquinolones are a class of compounds known in literature for their pharmacological properties. In recent years, many efforts were made on improving their synthesis, but no one tested the ability of these compounds as photoinitiators.

### AIMS OF THE INVENTION

It is a first aim of the invention to provide for novel photocurable compositions comprising ketoquinolones.

It is a further aim of the invention to provide for novel ketoquinolones, their use as photoinitiators and photocurable compositions comprising them.

Is a further aim of the invention to provide for a process for photocuring ethylenically unsaturated compounds using ketoquinolones, as well as articles of manufacture made by said process.

### DESCRIPTION OF THE INVENTION

We surprisingly discovered that a class of ketoquinolones is able to generate radicals under light irradiation. Moreover, under appropriate substitutions we were able to achieve the photopolymerization also under LED, that means that we were able to tune the absorption properties of this class of compounds without affecting the reactivity. The new compounds were tested in different formulations and compared to well know commercial photoinitiators demonstrating their good performance. So, we unexpectedly found that specific ketoquinolones well react to light sources in a range between 200 to 800 nm, preferably in a range between 200 to 500 nm , and more preferably, react to LED sources emitting in the range from 350 to 420 nm, representing a novelty compared to prior art. Therefore, the present invention relates to specific ketoquinolones useful as photoinitiators, compositions comprising said photoinitiators and a process for the photopolymerization of compositions comprising said ketoquinolones.

According to one of its aspects, the present invention relates to a photocurable composition comprising:
a) from 50 to 99.9%, preferably from 70 to 98.9% by weight, based on the total weight of the composition, of at least one ethylenically unsaturated compound; and
b) from 0.1 to 35%, preferably from 0.1 to 20%, and more preferably from 0.2 to 15% by weight, based on the total weight of the composition, of at least one compound of formula (I) (A) to (D): 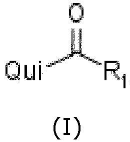 wherein:
   R₁ is a substituted or unsubstituted C1-C20 alkyl group, a substituted or unsubstituted aryl or a substituted or unsubstituted heteroaryl.
   Qui is a quinolone group of formula: 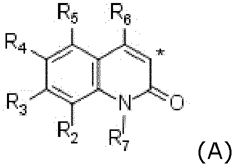 wherein:
      R₂, R₃, R₄ and R₅, independently of one another are selected from hydrogen, substituted or unsubstituted C1-C20 alkyl, -N(C1-C6 alkyl)₂, piperidino, morpholino, piperazino, -O-Rs, -S-R₈, -O-[CH₂]ₙ-COOR₈ and -S-[CH₂]ₙ-COORs; n=1-8 and R₈ is selected from hydrogen, substituted or unsubstituted C1-C20 alkyl, C1-C50 alkyl which is interrupted by one or more oxygens and which may terminate with a hydroxy group, substituted or unsubstituted C2-C12 alkenyl, substituted or unsubstituted aryl, substituted or unsubstituted heteroaryl and C5-C6 cycloalkyl;
      R₆ is hydrogen, a hydroxy group or a C1-C4 alkyl group;
      R₇ is hydrogen or a C1-C10 alkyl group;
      or Qui is a substituted or unsubstituted benzo-quinolone group of formula: 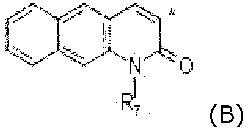 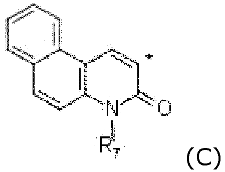 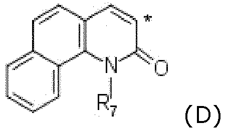 wherein R₇ is hydrogen or a C1-C10 alkyl group and the star indicates the carbon atom which is bound to the keto group of formula (I).

According to the present invention, the terms "photocuring" and "photopolymerizing" and related terms, are synonyms.

The expression "based on the total weight of the composition" herein means that the % weight amounts of the compounds and of the additional components of the composition are calculated on the sum of the weights of said compounds and of said additional components, irrespective of the fact that water and/or solvents may be present in the composition.

According to another of its aspects, the present invention relates to a compound of formula (Ia): 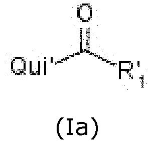 wherein:
R'₁ is a substituted or unsubstituted aryl or a substituted or unsubstituted heteroaryl.
Qui' is a quinolone group of formula: 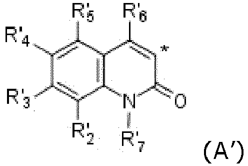 wherein:
   R'₂, R'₃, R'₄ and R'₅ independently of one another are selected from substituted or unsubstituted C2-C20 alkyl, -N(C1-C6 alkyl)₂, piperidino, morpholino, piperazino, -O-Rs, -S-R₈, -O-[CH₂]ₙ-COOR₈ and -S-[CH₂]ₙ-COORs; n=1-8 and R₈ is selected from hydrogen, substituted or unsubstituted C1-C20 alkyl, C1-C50 alkyl which is interrupted by one or more oxygens and which may terminate with a hydroxy group, substituted or unsubstituted C2-C12 alkenyl, substituted or unsubstituted aryl, substituted or unsubstituted heteroaryl and C5-C6 cycloalkyl;
   R'₆ is hydrogen or a C1-C4 alkyl group;
   R'₇ is a C1-C10 alkyl group;
   or Qui' is a substituted or unsubstituted benzo-quinolone group of formula (B'), (C') or (D'): 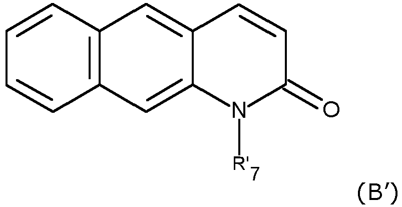 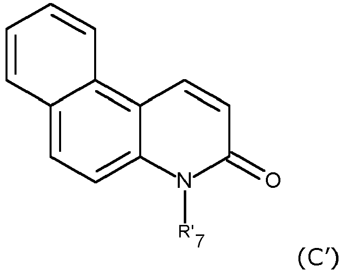 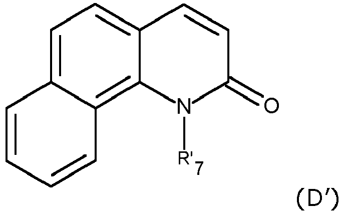 wherein
      R'₇ is a C1-C10 alkyl group and the star indicates the carbon atom which is bound to the keto group of formula (Ia).

According to another of its aspects, the present invention relates to a process for photopolymerizing which comprises:
(i) preparing or providing a photopolymerizable composition comprising (a) and (b), as above defined; and
(ii) photopolymerizing the composition of step (i) with a light source, emitting at wavelength comprised between 200 and 800 nm.

### DETAILED DESCRIPTION OF THE INVENTION

In the present description the expressions "alkyl" or "alkyl group" mean, where not differently indicated, a linear or branched, saturated alkyl chain containing the given number of carbon atoms and includes all possibility for each number of carbon atoms in the alkyl group, i.e. for three carbon atoms: n-propyl and isopropyl; for four carbon atoms: n-butyl, isobutyl and tertiary-butyl; for five carbon atoms: n-pentyl, 1,1-dimethyl-propyl, 2,2-dimethylpropyl and 2-methyl-butyl, etc..

"Alkenyl" or "alkenyl group" mean an unsaturated group containing from 2 to 12 carbon atoms, preferably C3 to C12 carbon atoms, which can be, for example, allyl, methallyl or undecenyl.

The expressions "cycloalkyl" or "cycloalkyl group" mean, where not differently indicated, an aliphatic ring containing 5 or 6 carbon atoms which can be cyclopentyl or cyclohexyl.

The expressions "aryl" or "aryl group" include, but it is not limited to, for example a substituted or unsubstituted phenyl group, a substituted or unsubstituted naphthyl group, an anthracenyl group, an indenyl group, a fluorenyl group.

The expressions "heteroaryl" or "heteroaryl group" include, but it is not limited to, for example a furan, a thiophene, a pyrrole, an oxazole, an isooxazole, a thiazole, an isothiazole, an imidazole, a pyrazole, a pyrane, a pyridine, a pyrrolidine, a piperidine, an indole, a quinoline, an isoquinoline, a xanthene, a carbazole, an acridine, an indeline, a julolidine and others.

The expression "C1-C50 alkyl which is interrupted by one or more oxygens" means that, in case more than one oxygen atom is present, said oxygen atoms are separated from one another by at least one methylene group, i.e. the oxygen atoms are non-consecutive. Preferably, oxygen atoms are separated by an ethylene or a n-propylene chain. Preferably 1 to 20 oxygen atoms are present, more preferably 2 to 18. Examples include the following: -O-CH₂-OCH₃, -O-CH₂CH₂-OCH₂CH₃, -O-[CH₂CH₂O]ᵥCH₃, -O-[CH₂CH₂O]ᵥOH, -O-[CH₂CH₂O]ᵥCH₂CH₃, -CH₂-O-[CH₂CH₂O]ᵥCH₃ with v=1-24, -O-[CH₂CH₂CH₂O]ₚOH, -O-[CH₂CH₂CH₂O]ₚCH₃, -O-[CH₂CH₂CH₂O]ₚCH₂CH₃, -CH₂-O-[CH₂CH₂CH₂O]ₚCH₃ with p=1-16.

When a group is substituted, the term "substituted" means that said group bears one or more substituents, said substituents being preferably selected from halogen atom, alkyl, cycloalkyl, alkoxy, alkylamino, dialkylamino, alkylthio or arylthio group, heterocyclic groups; preferably selected from methyl, ethyl, isopropyl, tert-butyl, phenyl, trifluoromethyl, cyano, acetyl, ethoxycarbonyl, carboxyl, carboxylate, amino, methylamino, dimethylamino, ethylamino, diethylamino, isopropylamino, diisopropylamino, cyclohexylamino, dicyclohexylamino, acetylamino, piperidino, pyrrolidyl, methoxy, ethoxy, propoxy, isopropoxy, butoxy, pentyloxy, phenoxy, hydroxyl, acetoxy, -PO₃H, methylthio, ethylthio, i-propylthio, n-propylthio, phenyltio, mercapto, acetylthio, thiocyano, methylsulfinyl, methylsulfonyl, dimethylsulfonyl, sulfonate groups, fluorine atom, chlorine atom, bromine atom, iodine atom, trimethylsilyl, triethylsilyl, trimethylstannyl, furyl, thienyl, pyridyl and morpholino. Among said substituents, electron donating groups such as alkoxy groups, for example methoxy, ethoxy, isopropoxy, tert-butoxy or phenoxy groups, methyl, ethyl, isopropyl, hydroxyl, acetoxy, benzoyloxy groups, etc. as well as a thioalkyl group, such as methylthio, ethylthio, n-propylthio, i-propylthio, butylthio, pentylthio, or a arylthio group, such as phenylthio, are preferred.

Unless expressly otherwise specified, herein all the % are weight percentage.

According to an embodiment, in formula (I) of the present description R₂, R₃, R₄ and R₅ are selected from substituted or unsubstituted C2-C20 alkyl. According to preferred embodiments, in formula (I) of the present description, at least one of the following conditions are preferred:
- R₁ is a substituted or unsubstituted aryl group, preferably a phenyl group, more preferably an aryl group substituted with a C1-C20 alkyl group, preferably a phenyl group substituted with a C1-C20 alkyl group;
- Qui is a quinolone group of formula (A) in which at least one of R₂, R₃, R₄ and R₅ is different from hydrogen, more preferably a quinolone group of formula (A) in which at least one of R₂, R₃, R₄ and R₅ is -O-Rs or -S-R₈ and R₈ is a C1-C20 alkyl; most preferably a quinolone group of formula (A) in which R₃ is -O-Rs or -S-R₈ and R₈ is a C1-C20 alkyl;
- R₆ is hydrogen;
- R₇ is a C1-C8 alkyl.

According to a preferred embodiment, two, three or all the above conditions are simultaneously met.

In another preferred embodiment, in the compounds of formula (I), Qui is a quinolone group of formula (A), in which R₆ is hydrogen, at least two of R₂, R₃, R₄ and R₅ are -O-Rs group and R₈ is a C1-C20 alkyl group. According to preferred embodiments, in formula (Ia) of the present description, at least one of the following conditions are preferred:
- R'₁ is a substituted or unsubstituted aryl group, preferably a phenyl group, more preferably an aryl group substituted with a C1-C20 alkyl group, preferably a phenyl group substituted with a C1-C20 alkyl group;
- Qui' is a quinolone group of formula (A') in which at least one of R'₂, R'₃, R'₄ and R'₅ is -O-Rs or -S-R₈ and R₈ is a C1-C20 alkyl;
- R'₆ is hydrogen;
- R'₇ is a C1-C8 alkyl.

According to a preferred embodiment, two, three or all the above conditions are simultaneously met.

In another preferred embodiment, in the compounds of formula (Ia), Qui' is a quinolone group of formula (A'), in which R'₆ is hydrogen, at least two of R'₂, R'₃, R'₄ and R'₅ are -O-Rs group and R₈ is a C1-C20 alkyl group, preferably a C1-C10 alkyl group.

The compounds represented by formulas (I) or (Ia) can be prepared according conventional methods known to the skilled in the art as reported, for example, in Eur. J. Org. Chem (2018) 896-900.

According to the invention, the photoinitiators of formulas (I) or (Ia) can be used to prepare photocurable compositions comprising ethylenically unsaturated compounds (a).

Said unsaturated compounds (a) can contain one or more olefinic double bonds. They can be low-molecular weight (monomeric) or high-molecular weight (oligomeric) compounds.

Examples of suitable low molecular weight monomers (monomeric compounds) having one double bond are alkyl or hydroxyalkyl acrylates or methacrylates, such as methyl-, ethyl-, butyl-, 2-ethylhexyl-,2-hydroxyethyl- or isobornyl-acrylate; and methyl or ethyl methacrylate. Further examples are resins modified with silicon or fluorine, e.g. silicone acrylates. Further examples of these monomers are acrylonitrile, acrylamide, methacrylamide, N-substituted (meth)acrylamides, styrene, alkylstyrenes and halogeno styrenes, vinyl esters such as vinyl acetate, vinyl ethers such as iso-butyl vinyl ether, N-vinylpyrrolidone, vinyl chloride or vinylidene chloride.

Examples of monomers having more than one double bond are the ethylene glycol diacrylate, propylene glycol diacrylate, neopentyl glycol diacrylate, hexamethylene glycol diacrylate, bisphenol A diacrylate, 4,4'-bis-(2-acryloyloxyethoxy)-diphenylpropane, trimethylolpropane triacrylate, pentaerythritol triacrylate or tetraacrylate, vinyl acrylate, divinyl benzene, divinyl succinate, diallyl phthalate, triallyl phosphate, triallyl isocyanurate or tris-(2-acryloylethyl) isocyanurate.

Examples of high-molecular weight (oligomeric) polyunsaturated compounds are acrylated epoxy resins, acrylated or vinyl-ether- or epoxy-group-containing polyesters, acrylated polyurethanes or acrylated polyethers. Further examples of unsaturated oligomers are unsaturated polyester resins which are usually prepared from maleic acid, phthalic acid and one or more diols and which have molecular weights of from about 500 Da to 3,000 Da. Such unsaturated oligomers can also be referred to as prepolymers.

Examples of compounds (a) which are particularly suitable for the implementation of the present invention, are esters of ethylenically unsaturated carboxylic acids and polyols or polyepoxides, and polymers containing ethylenically unsaturated groups in the chain or in side groups, e.g. unsaturated polyesters, polyamides and polyurethanes and copolymers thereof, alkyl resins, polybutadiene and butadiene copolymers, polyisoprene and isoprene copolymers, polymers and copolymers having (meth)acrylic groups in side chains, as well as mixtures thereof.

Illustrative examples of unsaturated carboxylic acids or anhydrides, useful for the preparation of the above esters, are acrylic acid, methacrylic acid, maleic anhydride, crotonic acid, itaconic acid, cinnamic acid and unsaturated fatty acids such as linolenic acid and oleic acid. Acrylic and methacrylic acid are preferred.

Examples of polyols, which can also be esterified, are aromatic and aliphatic and cycloaliphatic polyols, preferably aliphatic and cycloaliphatic polyols.

Aromatic polyols are, for example, hydroquinone, 4,4'-dihydroxydiphenyl, 2,2-di(4-hydroxyphenyl) propane, as well as novolaks and resoles. Polyepoxides, which can be esterified, include those based on the said polyols, especially the reaction products between aromatic polyols and epichlorohydrin. Also suitable as polyols are polymers and copolymers that contain hydroxyl groups in the polymer chain or in side groups, for example polyvinyl alcohol and copolymers thereof or polymethacrylic acid hydroxyalkyl esters or copolymers thereof. Further suitable polyols are oligoesters carrying hydroxyl terminal groups.

Examples of aliphatic and cycloaliphatic polyols include alkylenediols containing preferably from 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene glycol, polyethylene glycols having molecular weights of preferably from 200 Da to 1,500 Da, 1,3-cyclopentanediol, 1,2-, 1,3- or 1,4-cyclohexanediol, 1,4-dihydroxymethyl cyclohexane, glycerol, tris(β-hydroxy-ethyl)amine, trimethylolethane, trimethylolpropane, pentaerythritol, dipentaerythritol and sorbitol.

Further suitable ethylenically unsaturated compounds (a) are unsaturated polyamides obtained from unsaturated carboxylic acids and aromatic, aliphatic and cycloaliphatic polyamines having preferably from 2 to 6, preferably from 2 to 4, amino groups. Examples of such polyamines are: ethylenediamine, 1,2- or 1,3-propylenediamine, 1,2-, 1,3- or 1,4-butylenediamine, 1,5-pentylenediamine, 1,6-hexylenediamine, octylenediamine, dodecylene diamine, 1,4-diaminocyclohexane, isophoronediamine, phenylene diamine, bisphenylenediamine, di-(β-aminoethyl) ether, diethylene triamine, triethylenetetramine and di(β-aminoethoxy)- and di(β-aminopropoxy)ethane. Other suitable polyamines are polymers and copolymers which may contain additional amino groups in the side chain and oligoamides containing amino end groups.

Specific examples of such unsaturated polyamides are: methylenebisacrylamide, 1,6-hexamethylene bisacrylamide, diethylenetriamine trismethacrylamide, bis(methacrylamidopropoxy) ethane and N-[(β-hydroxyethoxy)ethyl]-acrylamide.

Unsaturated polyurethanes are also suitable for the implementation of the present invention as components (a), for example those derived from saturated or unsaturated diisocyanates and unsaturated or saturated diols.

Polybutadiene and polyisoprene and copolymers thereof may also be used. Suitable monomers include, for example, olefins, such as ethylene, propene, butene and hexene, (meth)acrylates, acrylonitrile, styrene and vinyl chloride.

Polymers having unsaturated (meth)acrylate groups in the side chain can also be used as component (a). They may typically be reaction products of epoxy resins based on novolak with (meth)acrylic acid; homo- or copolymers of vinyl alcohol or hydroxyalkyl derivatives thereof that have been esterified with (meth)acrylic acid; and homo- and co-polymers of (meth)acrylates that have been esterified with hydroxyalkyl (meth)acrylates.

The photocurable compositions of the present invention can also comprise one or more of the following components: (c) photosensitizers and/or (d) accelerators/coinitiators and/or further (e) photoinitiators and/or (f) additives, in addition to compounds (a) and (b).

The photocurable compositions of the present invention can also be formulated in compositions further comprising water and/or solvents, such as organic solvents.

Photosensitizers (c) can be present in an amount comprised between 0.01 and 15% by weight, based on the total weight of the composition, preferably between 0.01 and 10% by weight.

Examples of photosensitizers are those commonly used in the art, aromatic carbonyl compounds, e.g. benzophenones, thioxanthones, anthraquinones and 3-acylcoumarin derivatives, terphenyls, styryl ketones, and 3-(aroylmethylene)-thiazolines, camphorquinones and also eosin, rhodamine and erythrosine dyes.

Examples of thioxanthones are thioxanthone, 2-isopropyl thioxanthone, 2-chloro thioxanthone, 2-dodecyl thioxanthone, 2,4-diethyl thioxanthone, 2,4-dimethyl thioxanthone, 1-methoxycarbonyl thioxanthone, 2-ethoxycarbonyl thioxanthone, 3-(2-methoxyethoxycarbonyl) thioxanthone, 4-butoxycarbonyl thioxanthone, 3-butoxycarbonyl-7-methyl thioxanthone, 1-cyano-3-chloro thioxanthone, 1-ethoxycarbonyl-3-chloro thioxanthone, 1-ethoxycarbonyl-3-ethoxy thioxanthone, 1-ethoxycarbonyl-3-amino thioxanthone, 1-ethoxycarbonyl-3-phenylsulfuryl thioxanthone, 3,4-di[2-(2-methoxyethoxy)ethoxycarbonyl] thioxanthone, 1-ethoxycarbonyl-3-(1-methyl-1-morpholinoethyl) thioxanthone, 2-methyl-6-dimethoxymethyl thioxanthone, 2-methyl-6-(1,1-dimethoxybenzyl) thioxanthone, 2-morpholinomethyl thioxanthone, 2-methyl-6-morpholinomethyl thioxanthone, N-allylthioxanthone-3,4-dicarboximide, N-octylthioxanthone-3,4-dicarboximide, N-(1,1,3,3-tetramethylbutyl)-thioxanthone-3,4-dicarboximide, 1-phenoxy thioxanthone, 6-ethoxycarbonyl-1-2-methoxythioxanthone, 6-ethoxycarbonyl-2-methylthioxanthone, thioxanthone-2-polyethylene glycol ester, 2-hydroxy-3-(3,4-dimethyl-9-oxo-9H-thioxanthon-2-yloxy)-N,N,N-trimethyl-1-propanaminium chloride, or those described in the patent application PCT/EP2011/069514, such as n-dodecyl-7-methyl-thioxanthone-3-carboxylate and N,N-disobutyl-7-methyl-thioxanthone-3-carbamide. Also suitable are polymeric thioxanthone derivatives (e.g. Omnipol^{®} TX from IGM Resins B.V., Genopol^{®} TX-1 from Rahn A.G., Speedcure^{®} 7010 from Lambson Limited).

Example of benzophenones are benzophenone, 4-phenyl benzophenone, 4-methoxy benzophenone, 4,4'-dimethoxybenzophenone, 4,4'-dimethyl benzophenone, 4,4'-dichloro benzophenone, 4,4'-dimethylamino benzophenone, 4,4'-diethylamino benzophenone, 4-methyl benzophenone, 2,4,6-trimethyl benzophenone, 4-(4-methylthiophenyl) benzophenone, 3,3'-dimethyl-4-methoxy benzophenone, methyl 2-benzoyl benzoate, 4-(2-hydroxyethylthio) benzophenone, 4-(4-tolylthio) benzophenone, 4-benzoyl-N,N,N-trimethylbenzene methanaminium chloride, 2-hydroxy-3-(4-benzoylphenoxy)-N,N,N-trimethyl-1-propanaminium chloride monohydrate, 4-(13-acryloyl-1,4,7,10,13-pentaoxatridecyl) benzophenone, 4-benzoyl-N,N-dimethyl-N-[2-(1-oxo-2-propenyl)oxylethyl-benzene methanaminium chloride. Also suitable are polymeric benzophenone derivatives (e.g. Omnipol^{®} BP, Omnipol^{®} 2702 and Omnipol^{®} 682 all from IGM Resins B.V., Genopol^{®} BP-2 from Rahn A.G. and Speedcure^{®} 7005 from Lambson Limited).

Examples of 3-acylcoumarin derivatives are 3-benzoyl coumarin, 3-benzoyl-7-methoxy coumarin, 3-benzoyl-5,7-di(propoxy) coumarin, 3-benzoyl-6,8-dichloro coumarin, 3-benzoyl-6-chloro coumarin, 3,3'-carbonyl-bis[5,7-di(propoxy) coumarin], 3,3'-carbonyl-bis(7-methoxy coumarin), 3,3'-carbonyl-bis(7-diethylamino coumarin), 3-isobutyroyl coumarin, 3-benzoyl-5,7-dimethoxy coumarin, 3-benzoyl-5,7-diethoxy coumarin, 3-benzoyl-5,7-dibutoxy coumarin, 3-benzoyl-5,7-di(methoxyethoxy) coumarin, 3-benzoyl-5,7-di(allyloxy) coumarin, 3-benzoyl-7-dimethylamino coumarin, 3-benzoyl-7-diethylamino coumarin, 3-isobutyroyl-1,7-dimethylamino coumarin, 5,7-dimethoxy-3-(1-naphthoyl) coumarin, 5,7-dimethoxy-3(1-naphthoyl)-coumarin, 3-benzoylbenzo [f]coumarin, 7-diethylamino-3-thienoyl coumarin, 3-(4-cyanobenzoyl)-5,7-dimethoxy coumarin, or those described in EP2909243 and WO2017216699.

Examples of 3-(aroylmethylene) thiazolines are 3-methy-1,2-benzoylmethylene-β-naphtho thiazoline, 3-methyl-2-benzoylmethylene-benzo thiazoline, 3-ethyl-2-propionylmethylene-β-naphtho thiazoline. Examples of other aromatic carbonyl compounds are acetophenone, 3-methoxyacetophenone, 4-phenylacetophenone, benzyl, such as that described in WO 2013/164394, 2-acetylnaphthalene, 2-naphthaldehyde, 9,10-anthraquinone, 9-fluorenone, dibenzosuberone, xanthone, 2,5-bis(4-diethylaminobenzylidene) cyclopentanone, α-(para-dimethylamino benzylidene), ketones, such as 2-(4-dimethylamino-benzylidene)-indan-1-one or 3-(4-dimethylaminophenyl)-1-indan-5-yl-propenone, 3-phenylthiophthalimide, N-methyl-3,5-di(ethylthio)phthalimide.

Particularly preferred are thioxanthones and 3-acylcoumarins.

It was observed that the above components (c) increase the activity of photoinitiators (b) without shortening the shelf life of the compositions. Moreover, such compositions have the special advantage that an appropriate choice of the photosensitizer (c) allows the spectral sensitivity of photoinitiator (b) to be shifted to any desired wavelength region. The skilled in the art is able to select the suitable photosensitizer (c) to make the photoinitiator(s) (b) work at any desired wavelength region.

The accelerators/coinitiators (d), can be present in an amount comprised between 0.2 and 15% by weight, of the total weight of the composition, preferably from 0.2 to 8% by weight.

Examples of suitable accelerators/coinitiators are alcohols, thiols, thioethers, amines or ethers that have an available hydrogen, bonded to a carbon adjacent to the heteroatom, disulfides and phosphines, e.g. as described in EP 438 123 and GB 2 180 358.

Suitable examples of amine accelerators/co-initiators include, but are not limited to, aliphatic, cycloaliphatic, aromatic, aryl-aliphatic, heterocyclic, oligomeric or polymeric amines. They can be primary, secondary or tertiary amines, for example butyl amine, dibutyl amine, tributyl amine, cyclohexyl amine, benzyldimethyl amine, di-cyclohexyl amine, N-phenyl glycine, triethyl amine, phenyl-diethanol amine, triethanolamine, piperidine, piperazine, morpholine, pyridine, quinoline, esters of dimethylamino benzoic acid, Michler's ketone (4,4'-bis-dimethyl aminobenzophenone) and derivatives thereof.

As the amine accelerators/co-initiators, an amine-modified acrylate compound can be used; examples of such amine-modified acrylate include acrylates modified by reaction with a primary or secondary amine that are described in US 3,844,916, EP 280222, US 5,482,649 or US 5,734,002. Multifunctional amine and polymeric amine derivatives are also suitable as co-initiators some examples are Omnipol^{®} ASA from IGM Resins B.V., Genopol^{®} AB-2 from Rahn A.G., Speedcure^{®} 7040 from Lambson Limited or those described in US2013/0012611.

The further photoinitiators (e) can be present in an amount comprised between 0.5 and 15 % by weight, of the total weight of the composition, preferably between 1 and 10% by weight of the composition.

Examples of other suitable photoinitiators (e) are camphorquinone, benzophenone, benzophenone derivatives, acetophenone, acetophenone derivatives, dialkoxyacetophenones, α-hydroxyketones, α-aminoketones, 4-aroyl-1,3-dioxolanes, benzoin alkyl ethers and benzyl ketals, e.g. benzyl dimethyl ketal, ketosulfones, e.g 1-[4-[(4-benzoyl-phenyl)-thio]-phenyl]-2-methyl-2-[(4-methyl-phenyl)-sulfonyl]-propan-1-one (Esacure^{®} 1001, from IGM Resins B.V.), 3-ketocoumarins, for example as described in EP2909243 and WO2017216699, phenylglyoxylates and derivatives thereof, dimeric phenyl glyoxylates, peresters, e.g. benzophenonetetracarboxylic acid peresters, for example as described in EP 126 541, acylphosphine photoinitiators (which can be chosen among mono-acylphosphine oxides, bis-acylphosphine oxides, tris-acylphosphine oxides and multifunctional mono- or bisacylphosphine oxides), halomethyltriazines, hexaaryl bisimidazole/coinitiator systems, e.g. ortho-chlorohexaphenylbisimidazole in combination with 2-mercaptobenzothiazole, ferrocenium compounds or titanocenes, for example dicyclopentadienyl-bis(2,6-difluoro-3-pyrrolo-phenyl)titanium, O-acyloxime ester photoinitiators.

Examples of α-hydroxyketones and α-aminoketones are 1-hydroxy cyclohexylphenyl ketone, 2-hydroxy-2-methyl-1-phenyl-propane-1-one, 1-[4-(2-hydroxyethoxy)phenyl]-2-hydroxy-2-methyl-1-propane-1-one, 2-hydroxy-1-{4-[4-(2-hydroxy-2-methyl-propionyl)-benzyl]phenyl}-2-methyl-propane-1-one), 2-hydroxy-1-{4-[4-(2-hydroxy-2-methylpropionyl)-phenoxy]-phenyl}-2-methyl-propan-1-one, 2-methyl-1-(4-methylthiophenyl)-2-morpholinopropane-1-one), 2-benzyl-2-dimethylamino-1-(4-morpholinophenyl)-butane-1-one, and (2-(dimethylamino)-2-[(4-methylphenyl)methyl]-1-[4-(4-morpholinyl) phenyl]-1-butanone).

Examples of O-acyloxime ester photoinitiators are 1,2-octanedione,1-[4-(phenylthio)phenyl]-2-(O-benzoyloxime), ethanone 1-[9-ethyl-6-(2-methylbenzoyl)-9H-carbazole-3-yl] 1-(O-acetyloxime) or those described in GB 2339571.

Examples of acylphosphine photoinitiators include, but are not limited to, bis(2,4,6-trimethylbenzoyl)phenylphosphine oxide, bis(2,6-dimethoxybenzoyl)-2,4,4-trimethylpentyl phosphine oxide, bis(2,4,6-trimethylbenzoyl)-(2,4-dipentyloxyphenyl), 2,4,6-trimethylbenzoyl-diphenyl phosphine oxide and ethyl (2,4,6-trimethylbenzoyl) phenylphosphinate, Phenyl(2,4,6-trimethylbenzoyl)phosphinic acid, glycerol ethoxylated trimester (Omnipol^{®} TP from IGM Resins B.V.). Examples of the halomethyltriazines based photoinitiators are 2-[2-(4-methoxy-phenyl)-vinyl]-4,6-bis-trichloromethyl [1,3,5]triazine, 2-(4-methoxy-phenyl)-4,6-bis-trichloromethyl [1,3,5]triazine, 2-(3,4-dimethoxyphenyl)-4,6-bis-trichloromethyl [1,3,5]triazine, 2-methyl-4,6-bis-trichloromethyl [1,3,5] triazine.

Cationic photoinitiators can be also used as the further photoinitiators (e), when the photocurable compositions according to the invention are used in hybrid systems (which in this connection mean mixtures of free- radically and cationically curing systems). Examples of suitable cationic photoinitiators are aromatic sulfonium, phosphonium or iodonium salts, as described e.g. in US4,950,581, or cyclopentadienylarene-iron(II) complex salts, e.g. (η⁶-isopropylbenzene)(η⁵-cyclopentadienyl) iron(II) hexafluorophosphate or photolatent acids based on oximes, as described, for example, in GB 2 348 644, US4,450,598, US4,136,055, WO 00/10972 and WO 00/26219.

Additives (f) can be, for example, thermal initiators, binders, stabilizers, and mixture thereof.

The photocuring process according to the invention, especially in the case of pigmented compositions, may also be assisted by the addition, as additional additive (f), of a thermal initiator, a compound that forms free radicals when heated, e.g. an azo compounds, such as 2,2'-azobis(4-methoxy-2,4-dimethylvaleronitrile), a triazene, diazosulfide, pentazadiene or a peroxy compound, for example a hydroperoxide or peroxycarbonate, e.g. tert-butyl hydroperoxide, as described e.g. in EP 245 639.

Binders may also be added to the photocurable composition of the invention. The addition of binders is particularly advantageous when the photocurable compounds are liquid or viscous substances. The amount of binder may be, for example, from 5 to 60% by weight, preferably from 10 to 50% by weight, based on the total weight of the composition. The choice of binder is made in accordance with the field of use and the properties required therefor, such as developability in aqueous and organic solvent systems, adhesion to substrates and sensitivity to oxygen.

Suitable binders are, for example, polymers having a weight average molecular weight (Mw) of approximately from 5,000 Da to 2,000,000 Da, preferably from 10,000 Da to 1,000,000 Da. Illustrative examples are: homo- and copolymers of acrylates and methacrylates, e.g. copolymers of methyl methacrylate/ethyl acrylate/methacrylic acid, poly(methacrylic acid alkyl esters), poly(acrylic acid alkyl esters); cellulose esters and ethers, such as cellulose acetate, cellulose acetate butyrate, methylcellulose, ethylcellulose, polyvinylbutyral, polyvinylformal, cyclised rubber, polyethers such as polyethylene oxide, polypropylene oxide, polytetrahydrofuran, polystyrene, polycarbonates, polyurethanes, chlorinated polyolefins, e.g. polyvinyl chloride, co-polymers of vinyl chloride/vinylidene chloride, co-polymers of vinylidene chloride with acrylonitrile, methyl methacrylate and vinyl acetate, polyvinyl acetate, co-poly (ethylene/vinyl acetate), polymers such as polycaprolactam and poly(hexamethylene adipamide), polyesters such as poly(ethylene glycol terephthalate) and poly(hexamethylene glycol succinate).

Suitable stabilizers are, for example, thermal inhibitors, such as hydroquinone, hydroquinone derivatives, p-methoxyphenol, β-naphthol or sterically hindered phenols, e.g. 2,6-di(tert-butyl)-p-cresol, which prevent premature polymerization. In order to increase dark storage stability it is possible to use, for example, copper compounds, such as copper naphthenate, stearate or octoate, phosphorus compounds, for example triphenylphosphine, tributylphosphine, triethyl phosphite, triphenyl phosphite or tribenzyl phosphite, quaternary ammonium compounds, e.g. tetramethylammonium chloride or trimethylbenzylammonium chloride, or hydroxylamine derivatives, e.g. N,N-diethylhydroxylamine. For the purpose of excluding atmospheric oxygen during polymerization it is possible to add paraffin or similar wax-like substances which, being insoluble in the polymer, migrate to the surface at the beginning of the polymerization and form a transparent surface layer which prevents air from entering.

It is also possible to add a light stabilizer, such as UV absorbers, e.g. hydroxyphenylbenzotriazole, hydroxyphenylbenzophenone, oxalic acid amide or hydroxyphenyl-s-triazine type. Such components can be used on their own or in the form of mixtures, with or without the use of sterically hindered amines (HALS).

The photocurable compositions according to the invention may also comprise, as further additives (f), photoreducible dyes, e.g. a xanthene, benzoxanthene, benzothioxanthene, thiazine, pyronin, porphyrin or acridine dye, and/or radiation cleavable trihalomethyl compounds. These compounds are described, for example, in EP445624.

Further customary additives (f) are, depending upon the intended use, optical brighteners, fillers, pigments, both white and colored pigments, colorants, antistatics, wetting agents or flow improvers. Additives conventionally used in the art, e.g. antistatics, flow improvers and adhesion enhancers, can also be used.

It is also possible for chain-transfer reagents conventionally used in the art to be added to the compositions according to the invention. Examples are mercaptans, amines and benzothiazole.

The composition of the invention may also comprise colorants and/or colored pigments. Depending upon the intended use, both inorganic and organic pigments may be used. Such additives are well known to the person skilled in the art; some examples are carbon black, iron oxides, such as iron oxide yellow, iron oxide red, chromium yellow, chromium green, nickel titanium yellow, ultramarine blue, cobalt blue, bismuth vanadate, cadmium yellow and cadmium red. Examples of organic pigments are mono- or bis-azo pigments, and also metal complexes thereof, phthalocyanine pigments, polycyclic pigments, e.g. perylene, anthraquinone, thioindigo, quinacridone or triphenylmethane pigments, and also diketo-pyrrolo-pyrrole, isoindolinone, e.g. tetrachloroisoindolinone, isoindoline, dioxazine, benzimidazolone and quinophthalone pigments. The pigments may be used in the formulations on their own or in admixture.

Depending upon the intended use, the pigments can be added to the formulations in amounts conventionally used in the art, for example in an amount from 0.1 to 30% by weight or from 10 to 25% by weight, based on the total weight of the composition.

The composition may also comprise, for example, organic colorants of an extremely wide variety of classes. Examples are azo dyes, methine dyes, anthraquinone dyes and metal complex dyes. Usual concentrations are, for example, from 0.1 to 20% wt, especially from 1 to 5% wt, based on the total weight of the composition.

The choice of additives is governed by the field of use in question and the properties desired for that field. The additives (f) described above are known in the art and are accordingly used in the amounts conventionally used in the art.

The photocurable compositions of the invention may comprise water.

The photocurable compositions of the invention are suitable for various purposes, for example as a printing ink, such as screen printing inks, flexographic printing inks, offset printing inks and inkjet printing inks, as clearcoats, as colored coats, for example for wood or metal, as powder coatings, as coating materials inter alia for paper, wood, metal or plastics, as daylight-curable paints for marking structures and roads, for photographic reproduction processes, for holographic recording materials, for image-recording processes or in the production of printing plates that can be developed using organic solvents or using aqueous-alkaline media, for the production of masks for screen printing, as dental filling compounds, as adhesives, as pressure-sensitive adhesives, as laminating resins, as photoresists, e.g. galvanoresists, as etch resists or permanent resists, both liquid and dry films, as photostructurable dielectrics, and as solder masks for electronic circuits, as resists in the production of color filters for any type of display screen or in the creation of structures during the manufacture of plasma displays and electroluminescent displays, in the production of optical switches, optical gratings (interference gratings), in the manufacture of three-dimensional articles by bulk curing (UV curing in transparent moulds) or according to the stereolithography process, as described, for example, in US4,575,330, in the manufacture of composite materials (e.g. styrene polyesters which may include glass fibers and/or other fibers and other adjuvants) and other methods of printing in three dimensions well-known to one skilled in the art, in the coating or sealing of electronic components or as coatings for optical fibers.

The photocurable compositions of the invention are also suitable for the production of optical lenses, e.g. contact lenses or Fresnel lenses, in the manufacture of medical apparatus, aids or implants, in dry film paints. The photocurable compositions of the invention are also suitable for the preparation of gels having thermotropic properties. Such gels are described for example in DE 19700064 and EP 678534.

Any article comprising a compound of formulas (I) or (Ia) or a photocurable compositions of the invention represents a further subject-matter of the invention.

The compounds and compositions according to the invention may also be used as free-radical photoinitiators or photoinitiating systems for radiation-curable powder coatings.

The photocurable compositions according to the invention are suitable, for example, as coating materials for all kinds of substrate, for example wood, textiles, paper, ceramics, glass, plastics, such as polyesters, polyethylene terephthalate, polyolefins and cellulose acetate, especially in the form of films, and also metals, such as Al, Cu, Ni, Fe, Zn, Mg or Co and GaAs, Si or SiOz, to which a protective layer is to be applied or an image is to be applied e.g. by imagewise exposure.

According to another of its aspects, it is a further subject-matter of the invention a process for photocuring photopolymerizable compositions and inks, which process comprises:
(i) preparing or providing a photopolymerizable composition comprising:
   - compounds (a) and (b); as defined above; or
   - compounds (a), (b) as defined above, and one or more components selected from components (c), (d), (e) and (f) as defined above;
(ii) photopolymerizing the composition of step I with a light source.

According to a preferred embodiment, the photopolymerizable composition used in step (i) above comprises at least (a), (b) and (d).

A large number of the most varied kinds of light source may be used, the light source emitting at wavelengths from approximately 200 nm to approximately 800 nm. Both point sources and planiform radiators (lamp carpets) are suitable. Examples are: carbon arc lamps, xenon arc lamps, medium pressure, high pressure and low pressure mercury arc radiators, doped, where appropriate, with metal halides (metal halide lamps), microwave-excited metal vapour lamps, excimer lamps, superactinic fluorescent tubes, fluorescent lamps, argon incandescent lamps, flash lamps, photographic floodlight lamps, light-emitting diodes (LED), electron beams, X-rays and lasers.

According to one embodiment, said light source comprises UV light in at least one of the UVA, UVB and UVC ranges.

According to a preferred embodiment, said light source is a LED source, particularly preferred are LED light source emitting at wavelengths comprised between 365 nm and 420 nm, more preferably at 365 nm, 385 nm and 395 nm.

According to the invention the distance between the lamp and the substrate to be exposed may vary according to the intended use and the type and strength of the lamp, e.g. from 0.1 cm to 150 cm, preferably from 1 cm to 50 cm.

Said photopolymerizable composition may also be applied over a substrate already comprising a coated or printed layer. Said photopolymerizable composition may, after photopolymerization with said light source, be overprinted or overcoated with one or more compositions suitable for printing or coating.

The article obtained by applying said photopolymerizable composition to said substrate by said means of coating or printing, and photopolymerizing by said light source, with or without further elaboration of the article by further coating or printing, represents a further subject-matter of this invention.

As said, we surprisingly found that compounds of formulas (I) and (Ia) are effective as photoinitiatiors and their activity was measured for the first time. Moreover, we found that compounds of formulas (I) and (Ia) are very reactive with LED lamps both in clear and pigmented systems.

The invention is illustrated in detail below by the following examples, which are illustrative and not limiting.

In case of inconsistencies between the chemical name and the chemical structure herein indicated, the chemical structure prevails.

The wavy bond 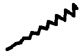 means that both cis and trans isomerism is possible.

### EXAMPLES

1H NMR spectra were recorded with a Bruker Avance 400 MHz or a Bruker DMX 500 MHz or a Bruker DMX 600 MHz.

Infrared spectra were recorded with a FT-IR 430 - Jasco.

### Example 1

### Synthesis of

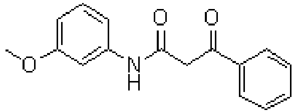

19.22 g (156.06 mmoles) of m-Anisidine and 25.00 g (130.07 mmoles) of Ethyl benzoylacetate were dissolved in 75 mL of N,N-Dimethylformamide. The reaction mixture was stirred at 150°C for 4 hours eliminating ethanol by distillation. Progress of the reaction was monitored by TLC. After completion of the reaction, the mixture was allowed to cool, poured into 625 mL of 6M hydrochloric acid and extracted with 375 mL of diethyl ether.

The organic layer was washed with 375 mL (x2) of water, dried over anhydrous sodium sulfate, filtered and the solvent removed by distillation under vacuum obtaining 27.92 g of a yellow oil (yield 80%).

¹H-NMR (CDCl₃, δ ppm): 3.80 (s, 3H), 4.10 (s, 2H), 6.68 (d, 1H), 7.08 (d, 1H), 7.22 (t, 1H), 7.31 (s, 1H), 7.51 (t, 2H), 7.64 (t, 1H), 8.02 (d, 2H), 9.30 (br s, 1H).

### Example 2

### Synthesis of

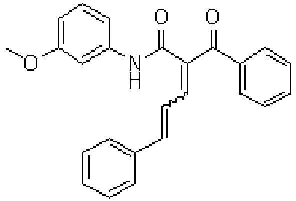

To a warm solution of 22.06 g (81.92 mmoles) of Example 1 and 10.83 g (81.95 mmoles) of trans-Cinnamaldehyde in 220 mL of toluene were added in sequence 0.698 g (8.197 mmoles) of piperidine, 0.492 g (8.193 mmoles) of acetic acid and 11.03 g of anhydrous sodium sulfate. The reaction mixture was refluxed for 1 hour under nitrogen atmosphere. Progress of the reaction was monitored by TLC. After completion of the reaction, the mixture was allowed to cool, poured into 300 mL of brine and extracted with 220 mL of ethyl acetate. The organic layer was washed with 300 mL (x3) of water, dried over anhydrous sodium sulfate, filtered and the solvent removed by distillation under vacuum obtaining 31.40 g of crude as a mixture of two regioisomers.

The product obtained was dissolved in 310 mL od anhydrous Tetrahydrofuran, cooled at 0°C, and 9.66 g (86.09 mmoles) of Potassium tert-butoxide were slowly added in portions under stirring. After stirring for 10 minutes, 12.81 g (90.25 mmoles) of Iodomethane were cautiously added to the reaction. Then the resulting mixture was stirred at room temperature for 1.5 hours under nitrogen atmosphere. Progress of the reaction was monitored by TLC. After completion of the reaction, the mixture was poured into 150 mL of ammonium chloride saturated solution, diluted with 150 mL of water and extracted with 300 mL of ethyl acetate. The organic layer was washed with 300 mL (x2) of water, dried over anhydrous sodium sulfate, filtered and the solvent removed by distillation under vacuum obtaining 32.12 g of crude. The crude was used in the next step without any further purification.

### Example 3

### Synthesis of

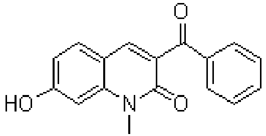

9.16 g (68.70 mmoles) of aluminum chloride were slowly added in portions under stirring to a solution of 4.55 g (11.45 mmoles as crude) of Example 2 in 135 mL of chlorobenzene. The resulting mixture was gradually heated to 120°C and then stirred at this temperature for 1.5 hours. Progress of the reaction was monitored by TLC. After completion of the reaction, the mixture was allowed to cool, poured into 450 mL of ice-water and extracted with 100 mL (x4) of dichloromethane/methanol (80:20). The organic layers were collected and the solvent removed by distillation under vacuum. The crude product was treated with 55 mL of toluene/cyclohexane (30:70), made to solidify and the solid washed under stirring at 100°C for 15 minutes. Then the mixture was allowed to cool and the solid recovered by filtration obtaining 2.56 g of an off-white solid (yield 80%).

¹H-NMR (DMSO-d₆, δ ppm): 3.55 (s, 3H), 6.82 (dd, 1H), 6.88 (d, 1H), 7.50 (t, 2H), 7.62 (t, 1H), 7.70 (d, 1H), 7.79 (d, 2H), 8.10 (s, 1H).

### Example 4

### Synthesis of

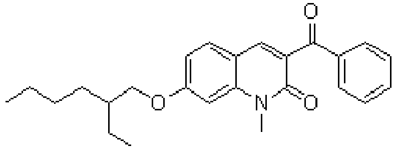

To a solution of 3.20 g (11.46 mmoles) of the product prepared in the Example 3 in 35 mL of N,N-Dimethylformamide were added in sequence 4.75 g (34.37 mmoles) of potassium carbonate, 0.34 g (2.27 mmoles) of sodium iodide and 4.43 g (22.94 mmoles) of 2-Ethylhexyl bromide. The reaction mixture was stirred at 80°C for 8 hours. Progress of the reaction was monitored by TLC. After completion of the reaction, the mixture was allowed to cool, poured into 200 mL of water and extracted with 100 mL of toluene. The organic layer was washed with 100 mL (x3) of water, dried over anhydrous sodium sulfate, filtered and the solvent removed by distillation under vacuum. The crude product was purified by flash column chromatography on silica gel (petroleum ether/ethyl acetate 80:20) followed by crystallization from cyclohexane obtaining 2.50 g of a white solid (yield 56%).

¹H-NMR (CDCl₃, δ ppm): 0.90-0.98 (m, 6H), 1.35 (m, 4H), 1.40-1.58 (m, 4H), 1.80 (m, 1H), 3.70 (s, 3H), 3.98 (m, 2H), 6.80 (d, 1H), 6.88 (dd, 1H), 7.43 (t, 2H), 7.55 (m, 2H),7.85 (d, 2H), 7.95 (s, 1H).

### Example 5

### Synthesis of

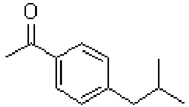

45.90 g (344.23 mmoles) of aluminum chloride were slowly added in portions under stirring to an ice-cooled solution of 44.00 g (327.82 mmoles) of Isobutylbenzene and 27.02 g (344.20 mmoles) of acetyl chloride in 200 mL of dichloromethane. The resulting mixture was stirred at room temperature for 1 hour under nitrogen atmosphere. After completion of the reaction, the mixture was poured into 800 mL of ice-water. The organic layer was separated, washed twice with water, dried over anhydrous sodium sulfate, filtered and the solvent removed by distillation under vacuum obtaining 57.45 g of a pale-yellow oil (yield 99%).

¹H-NMR (CDCl₃, δ ppm): 0.90 (d, 6H), 1.90 (m, 1H), 2.52 (d, 2H), 2.57 (s, 3H), 7.21 (d, 2H), 7.86 (d, 2H).

### Example 6

### Synthesis of

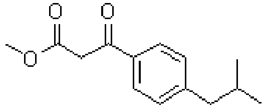

16.90 g (312.85 mmoles) of sodium methoxide were slowly added portionwise in 20 minutes under stirring at 90°C to a mixture of 50.00 g (283.67 mmoles) of Example 5 and 255.50 g (2836.37 mmoles) of dimethyl carbonate in 240 mL of toluene. The mixture was stirred at 90°C for 1 hour eliminating methanol by distillation. After stirring for 1 hour, additional 53.50 g (593.92 mmoles) of dimethyl carbonate in 50 mL of toluene were added to the mixture. Then the temperature was allowed to rise again to 90°C and additional 16.90 g (312.85 mmoles) of sodium methoxide were slowly added portionwise in 20 minutes. The mixture was stirred at 90°C for further 1 hour eliminating methanol by distillation. After completion of the reaction, the mixture was allowed to cool, poured into 600 mL of 12% hydrochloric acid and extracted with 200 mL of ethyl acetate. The organic layer was washed with 300 mL (x2) of water, dried over anhydrous sodium sulfate, filtered and the solvent removed by distillation under vacuum obtaining 66.52 g of a yellow oil (yield 100%).

¹H-NMR (CDCl₃, δ ppm): 0.89 (d, 6H), 1.90 (m, 1H), 2.52 (d, 2H), 3.73 (s, 3H), 3.98 (s, 2H), 7.23 (d, 2H), 7.85 (d, 2H).

### Example 7

### Synthesis of

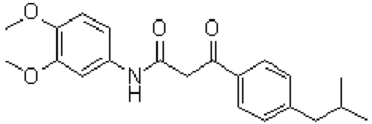

8.20 g (53.53 mmoles) of 3,4-Dimethoxyaniline and 11.40 g (48.66 mmoles) of Example 6 were dissolved in 120 mL of N,N-Dimethylformamide. The reaction mixture was stirred at 150°C for 3 hours eliminating methanol by distillation. Progress of the reaction was monitored by TLC. After completion of the reaction, the mixture was allowed to cool, diluted with 300 mL of toluene/ethyl acetate (60:40) and poured into 500 mL of 6M hydrochloric acid. The organic layer was separated and washed with 500 mL (x3) of water, dried over anhydrous sodium sulfate, filtered and the solvent removed by distillation under vacuum obtaining 17.00 g of a grey solid (yield 98%).

¹H-NMR (CDCl₃, δ ppm): 0.90 (d, 6H), 1.90 (m, 1H), 2.52 (d, 2H), 3.84 (s, 3H), 3.87 (s, 3H), 4.07 (s, 2H), 6.80 (d, 1H), 7.01 (dd, 1H), 7.27 (d, 2H), 7.30 (d, 1H), 7.93 (d, 2H), 9.25 (br s, 1H).

### Example 8

### Synthesis of

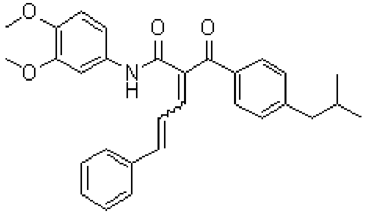

To a warm solution of 17.00 g (47.83 mmoles) of Example 7 and 6.32 g (47.82 mmoles) of trans-Cinnamaldehyde in 200 mL of toluene were added in sequence 0.407 g (4.78 mmoles) of piperidine, 0.287 g (4.78 mmoles) of acetic acid and 8.50 g of anhydrous sodium sulfate. The reaction mixture was refluxed for 1 hour under nitrogen atmosphere. Progress of the reaction was monitored by TLC. After completion of the reaction, the mixture was allowed to cool, poured into 400 mL of brine and extracted with 200 mL (x2) of dichloromethane. The organic layer was washed with 200 mL (x3) of water, dried over anhydrous sodium sulfate, filtered and the solvent removed by distillation under vacuum obtaining 22.40 g of crude as a mixture of two regioisomers. The crude was used in the next step without any further purification.

### Example 9

### Synthesis of

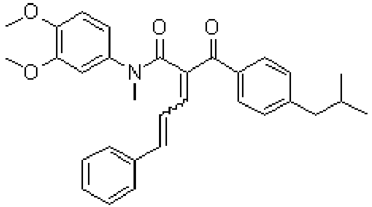

5.77 g (51.42 mmoles) of Potassium tert-butoxide were slowly added in portions under stirring to an ice-cooled solution of 22.40 g (47.70 mmoles as crude) of Example 8 in 300 mL of anhydrous Tetrahydrofuran. After stirring for 10 minutes, 7.65 g (53.90 mmoles) of Iodomethane were cautiously added to the reaction. Then the resulting mixture was stirred at room temperature for 1.5 hours under nitrogen atmosphere. Progress of the reaction was monitored by TLC. After completion of the reaction, the mixture was poured into 200 mL of ammonium chloride saturated solution, diluted with 200 mL of water and extracted with 300 mL of ethyl acetate. The organic layer was washed with 400 mL (x2) of water, dried over anhydrous sodium sulfate, filtered and the solvent removed by distillation under vacuum obtaining 23.00 g of crude that was used directly in the next step.

### Example 10

### Synthesis of

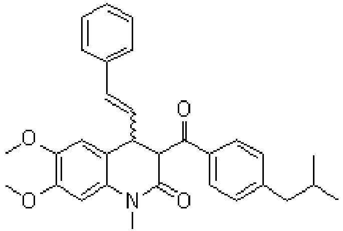

34.06 g (354.39 mmoles) of Methanesulfonic acid were slowly added under stirring to a solution of 23.00 g (47.56 mmoles as crude) of Example 9 in 460 mL of dichloromethane. After stirring for 10-15 minutes at room temperature, the reaction mixture was poured into 800 mL of water. The organic layer was separated and washed with 800 mL (x2) of water, dried over anhydrous sodium sulfate, filtered and the solvent removed by distillation under vacuum. The crude product was purified by flash column chromatography on silica gel (toluene/ethyl acetate 90:10) followed by crystallization from cyclohexane obtaining 10.11 g of an off-white solid (yield 44%).

¹H-NMR (CDCl₃, δ ppm): 0.90 (d, 6H), 1.90 (m, 1H), 2.52 (d, 2H), 3.46 (s, 3H), 3.83 (s, 3H), 3.95 (s, 3H), 4.15 (t, 1H), 4.68 (d, 1H), 6.22 (dd, 1H), 6.48 (d, 1H), 6.67 (s, 1H), 6.71 (s, 1H), 7.22-7.35 (m, 7H), 7.91 (d, 2H).

### Example 11

### Synthesis of

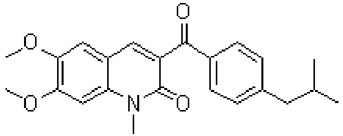

8.00 g (16.54 mmoles) of Example 10 were dissolved under vigorous stirring into 59.20 g (615.96 mmoles) of Methanesulfonic acid. The reaction mixture was stirred at room temperature for 4 hours. Progress of the reaction was monitored by TLC. After completion of the reaction, the mixture was poured into 800 mL of water and extracted with 300 mL of dichloromethane. The organic layer was washed in sequence with 300 mL of sodium hydrogen carbonate saturated solution and then with 300 mL of water, dried over anhydrous sodium sulfate, filtered and the solvent removed by distillation under vacuum. The crude product was purified by flash column chromatography on silica gel (toluene/ethyl acetate 65:35) followed by crystallization from cyclohexane/ethyl acetate (75:25) obtaining 1.85 g of a white-yellow solid (yield 29%).

¹H-NMR (CDCl₃, δ ppm): 0.90 (d, 6H), 1.90 (m, 1H), 2.52 (d, 2H), 3.74 (s, 3H), 3.93 (s, 3H), 4.04 (s, 3H), 6.80 (s, 1H), 7.00 (s, 1H), 7.20 (d, 2H), 7.79 (d, 2H), 7.88 (s, 1H).

### Example 12

### Synthesis of

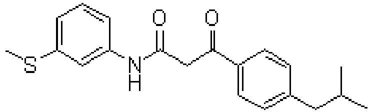

4.95 g (35.55 mmoles) of 3-(Methylthio)aniline and 7.57 g (32.31 mmoles) of Example 6 were dissolved in 80 mL of N,N-Dimethylformamide. The reaction mixture was stirred at 150°C for 4 hours eliminating methanol by distillation. Progress of the reaction was monitored by TLC. After completion of the reaction, the mixture was allowed to cool, poured into 400 mL of 6M hydrochloric acid and extracted with 300 mL of diethyl ether. The organic layer was washed with 300 mL (x2) of water, dried over anhydrous sodium sulfate, filtered and the solvent removed by distillation under vacuum obtaining 10.37 g of a yellow oil (yield 94%).

¹H-NMR (CDCl₃, δ ppm): 0.90 (d, 6H), 1.90 (m, 1H), 2.47 (s, 3H), 2.54 (d, 2H), 4.07 (s, 2H), 7.00 (d, 1H), 7.21 (t, 1H), 7.27 (d, 2H), 7.32 (d, 1H), 7.55 (s, 1H), 7.93 (d, 2H), 9.40 (br s, 1H).

### Example 13

### Synthesis of

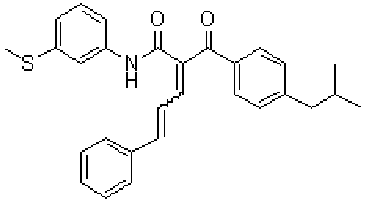

To a warm solution of 10.00 g (29.29 mmoles) of Example 12 and 3.87 g (29.28 mmoles) of trans-Cinnamaldehyde in 120 mL of toluene were added in sequence 0.249 g (2.92 mmoles) of piperidine, 0.175 g (2.91 mmoles) of acetic acid and 5.00 g of anhydrous sodium sulfate. The reaction mixture was refluxed for 1 hour under nitrogen atmosphere. Progress of the reaction was monitored by TLC. After completion of the reaction, the mixture was allowed to cool, poured into 250 mL of brine and extracted with 150 mL (x2) of dichloromethane. The organic layer was washed with 150 mL (x3) of water, dried over anhydrous sodium sulfate, filtered and the solvent removed by distillation under vacuum obtaining 13.00 g of crude as a mixture of two regioisomers. The crude was used in the next step without any further purification.

### Example 14

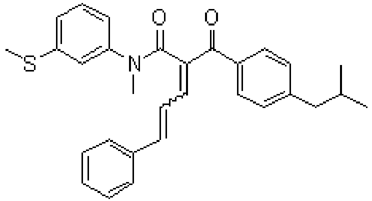

3.36 g (29.94 mmoles) of Potassium tert-butoxide were slowly added in portions under stirring to an ice-cooled solution of 13.00 g (28.53 mmoles as crude) of Example 13 in 180 mL of anhydrous Tetrahydrofuran. After stirring for 10 minutes, 4.45 g (31.35 mmoles) of Iodomethane were cautiously added to the reaction. Then the resulting mixture was stirred at room temperature for 1.5 hours under nitrogen atmosphere. Progress of the reaction was monitored by TLC. After completion of the reaction, the mixture was poured into 100 mL of ammonium chloride saturated solution, diluted with 100 mL of water and extracted with 200 mL of ethyl acetate. The organic layer was washed with 200 mL (x2) of water, dried over anhydrous sodium sulfate, filtered and the solvent removed by distillation under vacuum obtaining 13.40 g of crude. The crude was used in the next step without any further purification.

### Example 15

### Synthesis of

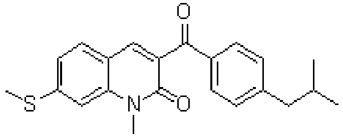

18.06 g (135.44 mmoles) of aluminum chloride were slowly added in portions under stirring to a solution of 10.60 g (22.57 mmoles as crude) of Example 14 in 300 mL of chlorobenzene. The resulting mixture was gradually heated to 120°C and then stirred at this temperature for 1 hour. Progress of the reaction was monitored by TLC. After completion of the reaction, the mixture was allowed to cool, poured into 1000 mL of ice-water and extracted with 300 mL dichloromethane. The organic layer was washed with 800 mL of water/brine (75:25), dried over anhydrous sodium sulfate, filtered and the solvent removed by distillation under vacuum. The crude product was purified by flash column chromatography on silica gel (toluene/ethyl acetate 90:10) followed by crystallization from cyclohexane/ethyl acetate (75:25) obtaining 1.07 g of a white solid (yield 13%).

¹H-NMR (CDCl₃, δ ppm): 0.94 (d, 6H), 1.91 (m, 1H), 2.55 (d, 2H), 2.63 (s, 3H), 3.75 (s, 3H), 7.15 (dd, 1H), 7.19 (d, 1H), 7.24 (d, 2H), 7.54 (d, 1H), 7.82 (d, 2H), 7.91 (s, 1H).

### Example 16

### Synthesis of

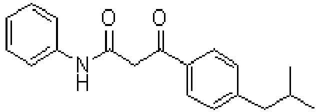

2.38 g (25.56 mmoles) of Aniline and 5.00 g (21.34 mmoles) of Example 6 were dissolved in 60 mL of N,N-Dimethylformamide. The reaction mixture was stirred at 150°C for 3.5 hours eliminating methanol by distillation. Progress of the reaction was monitored by TLC. After completion of the reaction, the mixture was allowed to cool, poured into 300 mL of 6M hydrochloric acid and extracted with 250 mL of diethyl ether. The organic layer was washed with 250 mL (x2) of water, dried over anhydrous sodium sulfate, filtered and the solvent removed by distillation under vacuum obtaining 5.97 g of an off-white solid (yield 95%).

¹H-NMR (CDCl₃, δ ppm): 0.90 (d, 6H), 1.90 (m, 1H), 2.54 (d, 2H), 4.07 (s, 2H), 7.10 (t, 1H), 7.25 (d, 2H), 7.35 (t, 2H), 7.60 (d, 2H), 7.95 (d, 2H), 9.35 (br s, 1H).

### Example 17

### Synthesis of

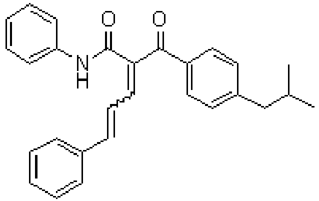

To a warm solution of 5.90 g (19.97 mmoles) of Example 16 and 2.64 g (19.98 mmoles) of trans-Cinnamaldehyde in 70 mL of toluene were added in sequence 0.170 g (2.00 mmoles) of piperidine, 0.120 g (2.00 mmoles) of acetic acid and 2.95 g of anhydrous sodium sulfate. The reaction mixture was refluxed for 1 hour under nitrogen atmosphere. Progress of the reaction was monitored by TLC. After completion of the reaction, the mixture was allowed to cool, poured into 175 mL of brine and extracted with 70 mL (x2) of dichloromethane. The organic layer was washed with 175 mL (x3) of water, dried over anhydrous sodium sulfate, filtered and the solvent removed by distillation under vacuum obtaining 8.17 g of crude as a mixture of two regioisomers. The crude was used in the next step without any further purification.

### Example 18

### Synthesis of

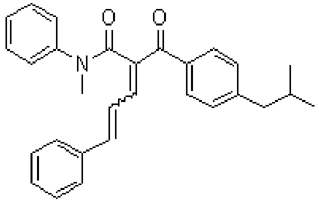

2.35 g (20.94 mmoles) of Potassium tert-butoxide were slowly added in portions under stirring to an ice-cooled solution of 8.17 g (19.95 mmoles as crude) of Example 17 in 125 mL of anhydrous Tetrahydrofuran. After stirring for 10 minutes, 3.11 g (21.91 mmoles) of Iodomethane were cautiously added to the reaction. Then the resulting mixture was stirred at room temperature for 1.5 hours under nitrogen atmosphere. Progress of the reaction was monitored by TLC. After completion of the reaction, the mixture was poured into 50 mL of ammonium chloride saturated solution, diluted with 100 mL of water and extracted with 150 mL of ethyl acetate. The organic layer was washed with 150 mL (x2) of water, dried over anhydrous sodium sulfate, filtered and the solvent removed by distillation under vacuum obtaining 8.45 g of crude. The crude was used in the next step without any further purification.

### Example 19

### Synthesis of

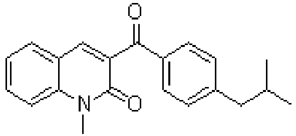

8.40 g (19.83 mmoles) of Example 18 were dissolved under vigorous stirring into 51.80 g (538.97 mmoles) of Methanesulfonic acid. The reaction mixture was stirred at 55°C for 1.5 hours. Progress of the reaction was monitored by TLC. After completion of the reaction, the mixture was poured into 600 mL of water and extracted with 300 mL of dichloromethane. The organic layer was washed with 300 mL (x2) of water, dried over anhydrous sodium sulfate, filtered and the solvent removed by distillation under vacuum. The crude product was purified by flash column chromatography on silica gel (toluene/ethyl acetate 85:15) followed by crystallization from cyclohexane/ethyl acetate (90:10) obtaining 2.58 g of a white solid (yield 41%).

¹H-NMR (DMSO-d₆, δ ppm): 0.88 (d, 6H), 1.90 (m, 1H), 2.54 (d, 2H), 3.67 (s, 3H), 7.29-7.38 (m, 3H), 7.63 (d, 1H), 7.71-7.78 (m, 3H), 7.85 (dd, 1H), 8.17 (s, 1H).

### Example 20

### Synthesis of

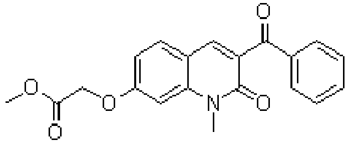

To a mixture of 3.50 g (12.53 mmoles) of Example 3 in 70 mL of N,N-Dimethylformamide were added 1.82 g (13.16 mmoles) of potassium carbonate. After stirring at room temperature for 10 minutes, 1.50 g (13.82 mmoles) of Methyl chloroacetate were added to the mixture. The reaction mixture was stirred at room temperature for 1.5 hours and then at 65°C for 1.5 hours. Progress of the reaction was monitored by TLC. After completion of the reaction, the mixture was allowed to cool and then poured into 250 mL of 1M hydrochloric acid. The resulting precipitate was removed by filtration, suspended in 35 mL of toluene and washed under stirring at 100°C for 15 minutes. Then the mixture was allowed to cool and the solid recovered by filtration obtaining 3.94 g of a white solid (yield 89%).

¹H-NMR (DMSO-d₆, δ ppm): 3.62 (s, 3H), 3.75 (s, 3H), 5.05 (s, 2H), 7.01 (dd, 1H), 7.06 (d, 1H),7.51 (t, 2H), 7.63 (t, 1H), 7.82 (m, 3H), 8.15 (s, 1H).

### Example 21

### Synthesis of

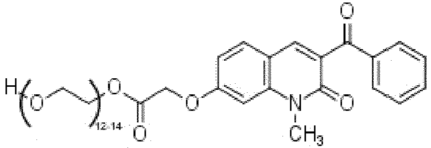

0.096 g (0.197 mmoles) of zirconium(IV) acetylacetonate were added under stirring to a warm mixture of 0.690 g (1.964 mmoles) of Example 20 and 2.946 g (4.910 mmoles) of polyethylene glycol 600 in 6.9 mL of toluene. The reaction mixture was stirred at 105°C for 2.5 hours eliminating methanol by distillation. Then additional 0.048 g (0.098 mmoles) of zirconium(IV) acetylacetonate were added and the reaction mixture was stirred at 105°C for further 1 hour eliminating methanol by distillation. Progress of the reaction was monitored by TLC. After completion of the reaction, the mixture was allowed to cool, dissolved in 30 mL of dichloromethane/EtOAc (50:50) and washed with 30 mL of 1M hydrochloric acid. The brown-dark precipitate obtained during the work-up was removed by filtration through a pad of celite and the phases were separated. The organic layer was washed with 30 mL (x4) of water, dried over anhydrous sodium sulfate, filtered and the solvent removed by distillation under vacuum obtaining 1.33 g of a light brown-yellow oil (yield 74%).

¹H-NMR (DMSO-d₆, δ ppm): 3.30-3.58 (m, 48H), 3.62-3.67 (m, 5H), 4.29 (m, 2H), 5.06 (s, 2H), 7.01 (dd, 1H), 7.06 (d, 1H), 7.51 (t, 2H), 7.65 (t, 1H), 7.80 (m, 3H), 8.15 (s, 1H).

### Example 22

### Synthesis of

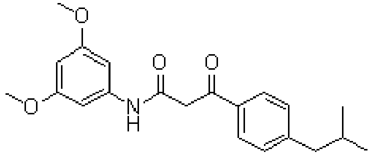

8.05 g (52.55 mmoles) of 3,5-Dimethoxyaniline and 11.20 g (47.80 mmoles) of Example 6 were dissolved in 100 mL of N,N-Dimethylformamide. The reaction mixture was stirred at 150°C for 4 hours eliminating methanol by distillation. Progress of the reaction was monitored by TLC. After completion of the reaction, the mixture was allowed to cool, poured into 500 mL of 6M hydrochloric acid and extracted with 250 mL of ethyl acetate/toluene (1:1). The organic layer was separated and washed with 300 mL (x3) of water, dried over anhydrous sodium sulfate, filtered and the solvent removed by distillation under vacuum obtaining 16.88 g of an off-white solid (yield 99%).

¹H-NMR (DMSO-d₆, δ ppm): 0.87 (d, 6H), 1.89 (m, 1H), 2.53 (d, 2H), 3.71 (s, 6H), 4.10 (s, 2H), 6.23 (t, 1H), 6.84 (d, 2H), 7.33 (d, 2H), 7.93 (d, 2H).

### Example 23

### Synthesis of

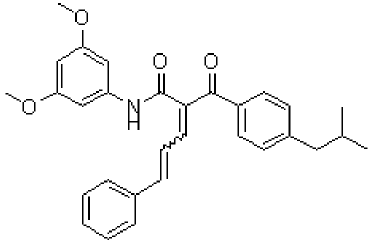

To a warm solution of 16.88 g (47.49 mmoles) of Example 22 and 6.28 g (47.52 mmoles) of trans-Cinnamaldehyde in 170 mL of toluene were added in sequence 0.405 g (4.76 mmoles) of piperidine, 0.285 g (4.75 mmoles) of acetic acid and 8.44 g of anhydrous sodium sulfate. The reaction mixture was refluxed for 1 hour under nitrogen atmosphere. Progress of the reaction was monitored by TLC. After completion of the reaction, the mixture was allowed to cool, poured into 200 mL of brine and extracted with 200 mL of ethyl acetate. The organic layer was washed with 200 mL (x3) of water, dried over anhydrous sodium sulfate, filtered and the solvent removed by distillation under vacuum obtaining 22.30 g of crude as a mixture of two regioisomers. The crude was used in the next step without any further purification.

### Example 24

### Synthesis of

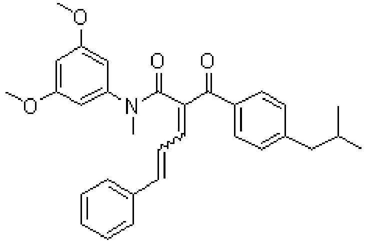

5.60 g (49.91 mmoles) of Potassium tert-butoxide were slowly added in portions under stirring to an ice-cooled solution of 22.30 g (47.49 mmoles as crude) of Example 23 in 223 mL of anhydrous Tetrahydrofuran. After stirring for 10 minutes, 7.41 g (52.21 mmoles) of Iodomethane were cautiously added to the reaction. Then the resulting mixture was stirred at room temperature for 1.5 hours under nitrogen atmosphere. Progress of the reaction was monitored by TLC. After completion of the reaction, the mixture was poured into 100 mL of ammonium chloride saturated solution, diluted with 100 mL of water and extracted with 150 mL of ethyl acetate. The organic layer was washed with 100 mL (x2) of water, dried over anhydrous sodium sulfate, filtered and the solvent removed by distillation under vacuum obtaining 22.26 g of crude. The crude was used in the next step without any further purification.

### Example 25

### Synthesis of

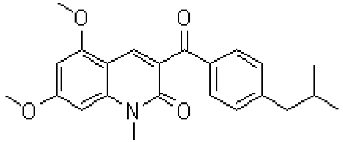

22.26 g (46.03 mmoles) of Example 24 were dissolved under vigorous stirring into 164.39 g (1710.44 mmoles) of methanesulfonic acid. The reaction mixture was stirred at room temperature for 24 hours. Progress of the reaction was monitored by TLC. After completion of the reaction, the mixture was poured into 500 mL of cold water and extracted with 250 mL of dichloromethane. The organic layer was washed in with 300 mL of water and brine, dried over anhydrous sodium sulfate, filtered and the solvent removed by distillation under vacuum. The crude product was purified by flash column chromatography on silica gel (toluene/ethyl acetate 75:25) followed by crystallization from cyclohexane/ethyl acetate (71:29) obtaining 1.71 g of a white solid (yield 10%).

¹H-NMR (DMSO-d₆, δ ppm): 0.88 (d, 6H), 1.88 (m, 1H), 2.53 (d, 2H), 3.62 (s, 3H), 3.93 (s, 3H), 3.97 (s, 3H), 6.54 (d, 1H), 6.62 (d, 1H), 7.28 (d, 2H), 7.70 (d, 2H), 8.14 (s, 1H).

### Comparative tests

The ketoquinolones of the invention, were compared with 4,4-Bis(diethylamino)benzophenone (COMP-1).

### Example 12.1

### Comparative tests

### Example 12.1.1.

### Clear Formulation

The photopolymerizable compositions for the test were prepared dissolving the photoinitiators and the co-initiator, Esacure^{®} EDB (IGM Resins B.V.) at a concentration of 3% by weight (wt) each in a mixture 99.5:0.5 wt of Ebecryl^{®} 605 and Ebecryl^{®} 350 (Allnex).

The photopolymerizable compositions placed in the sample lodgment of a FT-IR (FT-IR 430-Jasco), were exposed to a LED lamp (400 nm) located at a distance of 25 mm from the sample and at an angle of 30°. IR spectra were acquired at constant time intervals during the photopolymerization and the reduction over the time of the area of the peaks at 1408 and 810 cm⁻¹ assigned to the acrylic double bonds was determined using the IR software. This allows quantifying the degree of polymerization and therefore the efficiency of the photoinitiator.

The results expressed as % of polymerization over time, are reported in Table 1.

**Table 1.**

| **Photoinitiator** | **LED Lamp** | | **Hg Lamp** | |
|---|---|---|---|---|
| | **after 0.5"** | **after 2"** | **after 0.2"** | **After 1"** |
| COMP-1* | 23 | 44 | 0 | 2 |
| Example 4 | 51 | 57 | 56 | 66 |
| Example 11 | 57 | 58 | 53 | 60 |
| Example 15 | 62 | 73 | 65 | 72 |
| Example 19 | 37 | 47 | 51 | 61 |
| Example 25 | 60 | 67 | 59 | 68 |

| | | | | |
|---|---|---|---|---|
| * Comparative | | | | |

These tests confirm that compounds of formulas (I) and (Ia) are very reactive as photoinitiators.

### Example 12.1.2.

### Cyan Inkjet Ink LED Lamp (400 nm)

The photopolymerizable compositions for the test were prepared dissolving the photoinitiators and the co-initiator, Esacure^{®} EDB (IGM Resins B.V.) at a concentration of 5% by weight (wt) each in a Cyan Inkjet Ink.

The photopolymerizable compositions placed in the sample lodgment of a FT-IR (FT-IR 430-Jasco), were exposed to a LED lamp (400 nm) located at a distance of 25 mm from the sample and at an angle of 30°. IR spectra were acquired at constant time intervals during the photopolymerization and the reduction over the time of the area of the peaks at 1408 and 810 cm⁻¹ assigned to the acrylic double bonds was determined using the IR software. This allows quantifying the degree of polymerization and therefore the efficiency of the photoinitiator.

The results expressed as % of polymerization over time, are reported in Table 2.

**Table 2.**

| **Photoinitiator** | **LED Lamp** | | **Hg Lamp** | |
|---|---|---|---|---|
| | **after 0.5"** | **after 2"** | **after 1"** | **After 3"** |
| COMP-1* | 38 | 63 | 6 | 11 |
| Example 4 | 59 | 73 | 51 | 83 |
| Example 11 | 56 | 72 | 27 | 27 |
| Example 15 | 72 | 82 | 49 | 86 |
| Example 19 | 56 | 66 | 9 | 13 |
| Example 25 | 58 | 73 | 59 | 87 |

| | | | | |
|---|---|---|---|---|
| *Comparative | | | | |

These tests confirm the high reactivity of compounds of formulas (I) and (Ia) also in pigmented systems.

## Claims

1. A photocurable composition comprising:
a) from 50 to 99.9%, preferably from 70 to 98.9% by weight, based on the total weight of the composition, of at least one ethylenically unsaturated compound; and
b) from 0.1 to 35%, preferably from 0.1 to 20%, and more preferably from 0.2 to 15% by weight, based on the total weight of the composition, of at least one compound of formula (I) 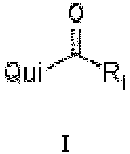 wherein:
R₁ is a substituted or unsubstituted C1-C20 alkyl group, a substituted or unsubstituted aryl or a substituted or unsubstituted heteroaryl.
Qui is a quinolone group of formula: 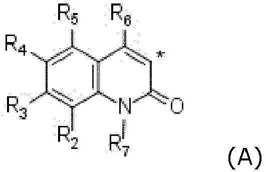 wherein:
R₂, R₃, R₄ and R₅ are, independently of one another, hydrogen, substituted or unsubstituted C1-C20 alkyl, -N(C1-C6 alkyl)₂, piperidino, morpholino, piperazino, -O-Rs, -S-R₈, -O-[CH₂]ₙ-COOR₈ or -S-[CH₂]ₙ-COOR₈ where n=1-8 and R₈ is hydrogen, substituted or unsubstituted C1-C20 alkyl, C1-C50 alkyl which is interrupted by one or more oxygens and which may terminate with a hydroxy group, C1-C20 alkyl, C2-C12 alkenyl, substituted or unsubstituted aryl, heteroaryl or C5-C6 cycloalkyl;
R₆ is hydrogen, a hydroxy group or a C1-C4 alkyl group;
R₇ is hydrogen or a C1-C10 alkyl group;
or Qui is a substituted or unsubstituted benzo-quinolone group of formula: 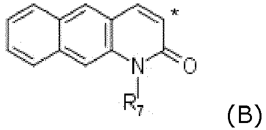 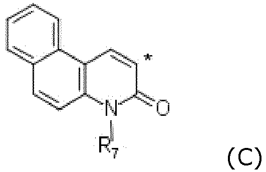 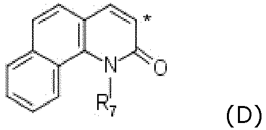 wherein R₇ is hydrogen or a C1-C10 alkyl group and the star indicates the carbon atom which is bound to the keto group of formula (I).

2. The photocurable composition of claim 1, wherein in formula (I) R₁ is a substituted or unsubstituted aryl group, a substituted or unsubstituted phenyl group.

3. The photocurable composition of claim 1 or 2 wherein in formula (I) Qui is a quinolone group of formula (A) in which at least one of R₂, R₃, R₄ and R₅ is different from hydrogen.

4. The photocurable composition of any one of claims 1 to 3, wherein in formula (I) R₆ is preferably hydrogen.

5. The photocurable composition of any one of claims 1 to 4, wherein in formula (I) R₇ is preferably a C1-C8 alkyl.

6. The photocurable composition of any one of claims 1 to 5, further comprising one or more of the following components:
c) from 0.01 to 15% by weight, based on the total weight of the composition, of one or more photosensitizer; and/or
d) from 0.2 to 15% by weight, based on the total weight of the composition, of accelerators/coinitiators; and/or
e) from 0.5 to 15% by weight, based on the total weight of the composition, of one or more further photoinitiators; and/or
f) conventional additives.

7. A photoinitiator of formula (Ia): 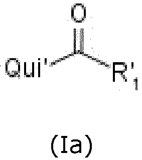 wherein:
R'₁ is a substituted or unsubstituted aryl or a substituted or unsubstituted heteroaryl;
Qui' is a quinolone group of formula: 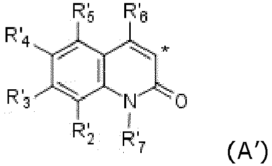 wherein:
R'₂, R'₃, R'₄ and R'₅ are, independently of one another are selected from hydrogen, substituted or unsubstituted C2-C20 alkyl, -N(C1-C6 alkyl)₂, piperidino, morpholino, piperazino, -O-Rs, -S-R₈, -O-[CH₂]ₙ-COOR₈ and -S-[CH₂]ₙ-COOR₈; n=1-8 and R₈ is selected from hydrogen, substituted or unsubstituted C1-C20 alkyl, C1-C50 alkyl which is interrupted by one or more oxygens and which may terminate with a hydroxy group, substituted or unsubstituted C2-C12 alkenyl, substituted or unsubstituted aryl, substituted or unsubstituted heteroaryl and C5-C6 cycloalkyl;
R'₆ is hydrogen or a C1-C4 alkyl group;
R'₇ is C1-C10 alkyl group;
or Qui' is a substituted or unsubstituted benzo-quinolone group of formula: 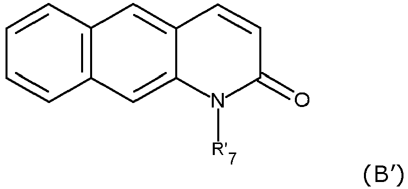 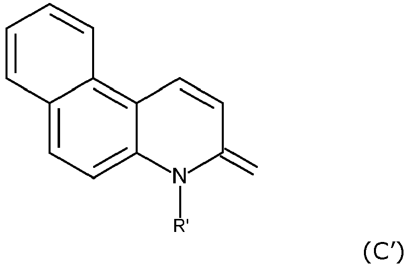 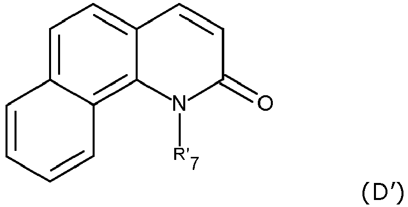 wherein R'₇ is C1-C10 alkyl group and the star indicates the carbon atom which is bound to the keto group of formula (Ia).

8. The compound of claim 7, wherein in formula (Ia) R'₁ is a substituted or unsubstituted aryl group and/or Qui' is a quinolone group of formula (A') in which at least one of R'₂, R'₃, R'₄ and R'₅ is -O-Rs or - S-Rs and R₈ is a C1-C20 alkyl.

9. The compound of any one of claim 7 or 8, wherein in formula (Ia) R'₆ is hydrogen and/or R'₇ is a C1-C8 alkyl

10. The compound of claim 7, wherein in formula (Ia) Qui' is a quinolone group of formula (A'), in which R'₆ is hydrogen, at least two of R'₂, R'₃, R'₄ and R'₅ are -O-Rs group and R₈ is a C1-C20 alkyl group.

11. A process for photocuring photopolymerizable compositions and inks, which comprises the following steps:
(i) preparing or providing a photopolymerizable composition comprising:
- compounds (a) and (b); as defined in claim 1 or
- compounds (a), (b) as defined in claim 6, and one or more components selected from components (c), (d), (e) and (f) as defined in claim 6, preferably (d);
(ii) photopolymerizing the composition of step (i) with a light source.

12. The process of claim 11 **characterized in that** said light source comprises UV light in at least one of the UVA, UVB and UVC ranges.

13. The process of claim 11 **characterized in that** said light source is a LED source emitting in the range from 350 to 420 nm.

14. The process of any one of claims 11 to 13, further comprising the step of applying said photopolymerizable composition to a substrate prior to photopolymerizing it.

15. An article of manufacture obtained according to the process of one of claims 11 to 14 or by three-dimensional printing of a mixture comprising the composition according to any one of claims 1 to 6.

## Patentansprüche

1. Eine photohärtbare Zusammensetzung, umfassend:
a) 50 bis 99,9 Gew.-%, vorzugsweise 70 bis 98,9 Gew.-%, bezogen auf das Gesamtgewicht der Zusammensetzung, mindestens einer ethylenisch ungesättigten Verbindung; und
b) 0,1 bis 35 Gew.-%, vorzugsweise 0,1 bis 20 Gew.-% und stärker bevorzugt 0,2 bis 15 Gew.-%, bezogen auf das Gesamtgewicht der Zusammensetzung, mindestens einer Verbindung der Formel (I) 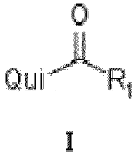 wobei:
R₁ eine substituierte oder unsubstituierte C1-C20-Alkylgruppe, ein substituiertes oder unsubstituiertes Aryl oder ein substituiertes oder unsubstituiertes Heteroaryl ist,
Qui eine Chinolongruppe der Formel: 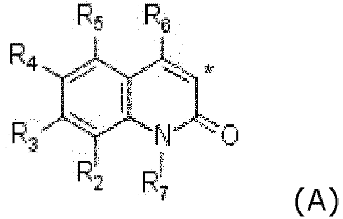 ist, wobei:
R₂, R₃, R₄ und R₅ unabhängig voneinander Wasserstoff, substituiertes oder unsubstituiertes C1-C20-Alkyl, -N(C1-C6-Alkyl)₂, Piperidino, Morpholino, Piperazino, -O-R₈, -S-R₈, -O-[CH₂]ₙ-COOR₈ oder -S-[CH₂]ₙ-COOR₈ sind, wobei n = 1-8 und R₈ Wasserstoff, substituiertes oder unsubstituiertes C1-C20-Alkyl, C1-C50-Alkyl, das durch ein oder mehrere Sauerstoffatome unterbrochen ist und mit einer Hydroxygruppe enden kann, C1-C20-Alkyl, C2-C12-Alkenyl, substituiertes oder unsubstituiertes Aryl, Heteroaryl oder C5-C6-Cycloalkyl ist;
R₆ Wasserstoff, eine Hydroxygruppe oder eine C1-C4-Alkylgruppe ist;
R₇ Wasserstoff oder eine C1-C10-Alkylgruppe ist;
oder Qui eine substituierte oder unsubstituierte Benzochinolongruppe der Formel: 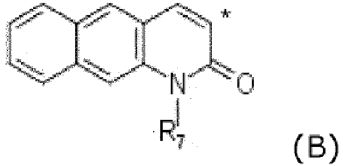 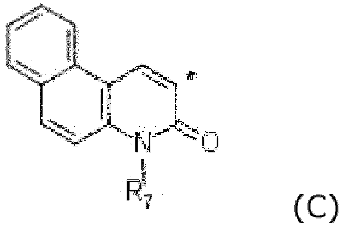 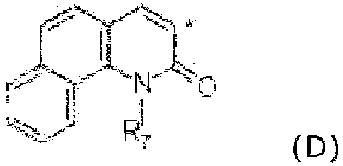
ist, wobei R₇ Wasserstoff oder eine C1-C10-Alkylgruppe ist und der Stern das Kohlenstoffatom anzeigt, das an die Ketogruppe der Formel (I) gebunden ist.

2. Die lichthärtbare Zusammensetzung nach Anspruch 1, wobei R₁ in Formel (I) eine substituierte oder unsubstituierte Arylgruppe, eine substituierte oder unsubstituierte Phenylgruppe ist.

3. Die photohärtbare Zusammensetzung nach Anspruch 1 oder 2, wobei Qui in Formel (I) eine Chinolongruppe der Formel (A) ist, in der mindestens eines von R₂, R₃, R₄ und R₅ von Wasserstoff verschieden ist.

4. Die lichthärtbare Zusammensetzung nach einem der Ansprüche 1 bis 3, wobei R₆ in Formel (I) vorzugsweise Wasserstoff ist.

5. Die lichthärtbare Zusammensetzung nach einem der Ansprüche 1 bis 4, wobei R₇ in Formel (I) vorzugsweise ein C1-C8-Alkyl ist.

6. Die photohärtbare Zusammensetzung nach einem der Ansprüche 1 bis 5, zusätzlich umfassend eine oder mehrere der folgenden Komponenten:
c) 0,01 bis 15 Gew.-%, bezogen auf das Gesamtgewicht der Zusammensetzung, eines oder mehrerer Photosensibilisatoren; und/oder
d) 0,2 bis 15 Gew.-%, bezogen auf das Gesamtgewicht der Zusammensetzung, Beschleuniger/Coinitiatoren; und/oder
e) 0,5 bis 15 Gew.-%, bezogen auf das Gesamtgewicht der Zusammensetzung, eines oder mehrerer weiterer Photoinitiatoren; und/oder
f) herkömmliche Zusatzstoffe.

7. Ein Photoinitiator der Formel (la): 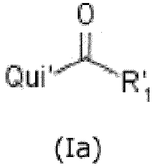 wobei:
R'₁ ein substituiertes oder unsubstituiertes Aryl oder ein substituiertes oder unsubstituiertes Heteroaryl ist;
Qui' eine Chinolongruppe der Formel: 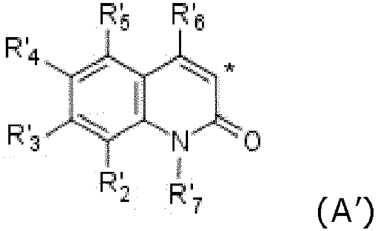 ist, wobei:
R'₂, R'₃, R'₄ und R'5 unabhängig voneinander aus Wasserstoff, substituiertem oder unsubstituiertem C2-C20-Alkyl, -N(C1-C6-Alkyl)₂, Piperidino, Morpholino, Piperazino, -O-Rs, -S- R₈, -O-[CH₂]ₙ-COOR₈ und -S- [CH₂]ₙ-COO R₈ ausgewählt ist; n = 1-8 und R₈ aus Wasserstoff, substituiertem oder unsubstituiertem C1-C20-Alkyl, C1-C50-Alkyl, das durch ein oder mehrere Sauerstoffatome unterbrochen ist und mit einer Hydroxygruppe enden kann, substituiertem oder unsubstituiertem C2-C12-Alkenyl, substituiertem oder unsubstituiertem Aryl, substituiertem oder unsubstituiertem Heteroaryl und C5-C6-Cycloalkyl ausgewählt ist;
R'₆ Wasserstoff oder eine C1-C4-Alkylgruppe ist;
R'₇ eine C1-C10-Alkylgruppe ist;
oder Qui' eine substituierte oder unsubstituierte Benzochinolongruppe der Formel: 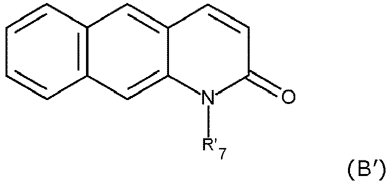 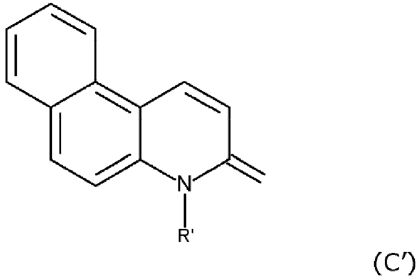 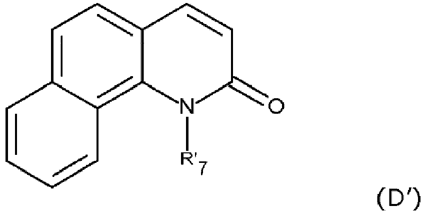
ist, wobei R'7 eine C1-C10-Alkylgruppe ist und der Stern das Kohlenstoffatom anzeigt, das an die Ketogruppe der Formel (la) gebunden ist.

8. Die Verbindung nach Anspruch 7, wobei in Formel (la) R'₁ eine substituierte oder unsubstituierte Arylgruppe ist und/oder Qui' eine Chinolongruppe der Formel (A') ist, in der mindestens einer der Reste R'₂, R'₃, R'₄ und R'₅ -O-Rs oder -S-R₈ ist und R₈ ein C1-C20-Alkyl ist.

9. Die Verbindung nach einem der Ansprüche 7 bis 8, wobei in Formel (la) R'₆ für Wasserstoff steht und/oder R'₇ ein C1-C8-Alkyl ist.

10. Die Verbindung nach Anspruch 7, wobei in Formel (la) Qui' eine Chinolongruppe der Formel (A') ist, in der R'₆ Wasserstoff ist, mindestens zwei von R'₂, R'₃, R'₄ und R'₅ -O-Rs-Gruppen sind und R₈ eine C1-C20-Alkylgruppe ist.

11. Ein Verfahren zur Lichthärtung von photopolymerisierbaren Zusammensetzungen und Tinten, das die folgenden Schritte umfasst:
(i) Herstellen oder Bereitstellen einer photopolymerisierbaren Zusammensetzung, umfassend:
- Verbindungen (a) und (b), wie in Anspruch 1 definiert, oder
- Verbindungen (a), (b), wie in Anspruch 6 definiert, und eine oder mehrere Komponenten, ausgewählt aus den Komponenten (c), (d), (e) und (f), wie in Anspruch 6 definiert, vorzugsweise (d);
(ii) Photopolymerisieren der Zusammensetzung aus Schritt (i) mit einer Lichtquelle.

12. Das Verfahren gemäß Anspruch 11, **dadurch gekennzeichnet, dass** die Lichtquelle UV-Licht in mindestens einem der Bereiche UVA, UVB und UVC umfasst.

13. Das Verfahren gemäß Anspruch 11, **dadurch gekennzeichnet, dass** die Lichtquelle eine LED-Quelle ist, die im Bereich von 350 bis 420 nm emittiert.

14. Das Verfahren gemäß einem der Ansprüche 11 bis 13, das außerdem den Schritt des Aufbringens der photopolymerisierbaren Zusammensetzung auf ein Substrat vor ihrer Photopolymerisierung umfasst.

15. Ein Gegenstand, hergestellt nach dem Verfahren gemäß einem der Ansprüche 11 bis 14 oder durch dreidimensionales Drucken einer Mischung, die die Zusammensetzung gemäß einem der Ansprüche 1 bis 6 enthält.

## Revendications

1. Composition photodurcissable comprenant :
a) de 50 à 99,9 %, de préférence de 70 à 98,9 % en poids, par rapport au poids total de la composition, d'au moins un composé éthylénique ment insaturé ; et
b) de 0,1 à 35 %, de préférence de 0,1 à 20 %, et plus préférentiellement de 0,2 à 15 % en poids, par rapport au poids total de la composition, d'au moins un composé de formule (I) 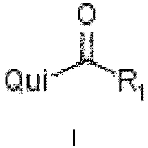 où :
R₁ est un groupe alkyle en C1-C20 substitué ou non substitué, un aryle substitué ou non substitué ou un hétéroaryle substitué ou non substitué.
Qui est un groupe quinolone de formule : 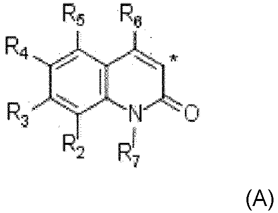 où :
R₂, R₃, R₄ et R₅ sont, indépendamment l'un de l'autre, de l'hydrogène, un alkyle en C1-C20 substitué ou non substitué, le -N(alkyle en C1-C6)₂, le piperidino, le morpholino, le piperazino, le -O-R8, le -S-R8, le -O-[CH₂]ₙ-COOR₈ ou le -S-[CH₂]ₙ-COOR₈ où n = 1-8 et R₈ est un hydrogène, un alkyle en C1-C20 substitué ou non substitué, un alkyle en C1-C50 interrompu par un ou plusieurs oxygènes et pouvant se terminer par un groupe hydroxy, un alkyle en C1-C20, un alcényle en C2-C12, un aryle substitué ou non substitué, un hétéroaryle ou un cycloalkyle en C5-C6 ;
R₆ est un hydrogène, un groupe hydroxy ou un groupe alkyle en C1-C4 ;
R₇ est un hydrogène ou un groupe alkyle de C1-C10 ;
ou Qui est un groupe benzo-quinolone substitué ou non substitué de formule : 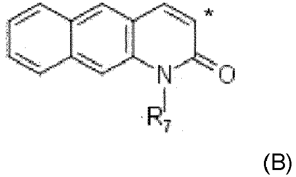 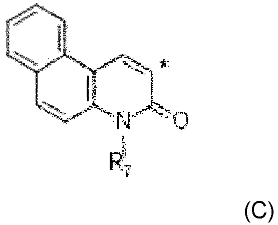 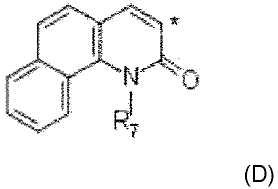 où R₇ est un hydrogène ou un groupe alkyle de C1-C10 et l'étoile indique l'atome de carbone qui est lié au groupe céto de formule I.

2. Composition photodurcissable de la revendication 1, dans laquelle, dans la formule (I), R₁ est un groupe aryle substitué ou non substitué, un groupe phényle substitué ou non substitué.

3. Composition photodurcissable de la revendication 1 ou 2 dans laquelle, dans la formule (I), Qui est un groupe quinolone de formule (A) dans laquelle au moins un élément parmi R₂, R₃, R₄ et R₅ est différent de l'hydrogène.

4. Composition photodurcissable de l'une quelconque des revendications 1 à 3, dans laquelle, dans la formule (I), R₆ est de préférence un hydrogène.

5. Composition photodurcissable de l'une quelconque des revendications 1 à 4, dans laquelle, dans la formule (I), R₇ est de préférence un alkyle en C1-C8.

6. Composition photodurcissable de l'une quelconque des revendications 1 à 5, comprenant en outre un ou plusieurs des composants suivants :
c) de 0,01 à 15 % en poids, par rapport au poids total de la composition, d'un ou plusieurs photosensibilisateurs ; et/ou
d) de 0,2 à 15 % en poids, par rapport au poids total de la composition, d'accélérateurs/coinitiateurs ; et/ou
e) de 0,5 à 15 % en poids, par rapport au poids total de la composition, d'un ou de plusieurs photo-initiateurs supplémentaires ; et/ou
f) les additifs conventionnels.

7. Photoinitiateur de formule (la) : 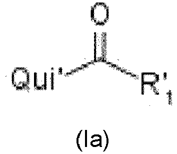 dans lequel :
R'₁ est un aryle substitué ou non substitué ou un hétéroaryle substitué ou non substitué ; Qui' est un groupe quinolone de formule : 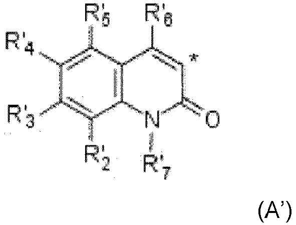 où :
R'₂, R'₃, R'₄ et R'₅ sont, indépendamment les uns des autres, choisis parmi l'hydrogène, l'alkyle en C2-C20 substitué ou non substitué, le -N(alkyle en C1-C6)₂, le piperidino, le morpholino, le piperazino, un -O-Rs, un -S-R₈, un -O-[CH₂]ₙ-COOR₈ and un -S-[CH₂]ₙ-COOR₈ ; n = 1-8 et R₈ est choisi parmi l'hydrogène, l'alkyle en C1-C20 substitué ou non substitué, l'alkyle en C1-C50 interrompu par un ou plusieurs oxygènes et pouvant se terminer par un groupe hydroxy, un alcényle C2-C12 substitué ou non substitué, un aryle substitué ou non substitué, un hétéroaryle substitué ou non substitué et un cycloalkyle C5-C6 ;
R'₆ est un hydrogène ou un groupe alkyle en C1-C4 ;
R'₇ est un groupe alkyle de C1-C10 ;
ou Qui' est un groupe benzo-quinolone substitué ou non substitué de formule : 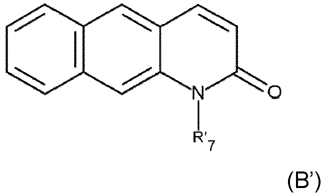 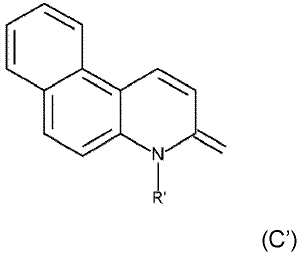 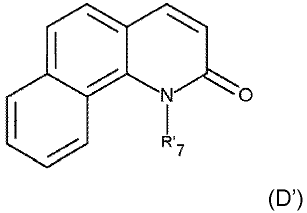
où R'₇ est un groupe alkyle en C1-C10 et l'étoile indique l'atome de carbone lié au groupe céto de la formule (la).

8. Composé de la revendication 7, dans lequel, dans la formule (Ia), R'₁ est un groupe aryle substitué ou non substitué et/ou Qui' est un groupe quinolone de formule (A') dans lequel au moins un élément parmi R'₂, R'₃, R'₄ et R'₅ est un -O-R₈ ou un -S-R₈ et R₈ est un alkyle en C1-C20.

9. Composé de l'une quelconque des revendications 7 ou 8, dans lequel, dans la formule (Ia), R'₆ est un hydrogène et/ou R'₇ est un alkyle en C1-C8

10. Composé de la revendication 7, dans lequel dans la formule (la) Qui' est un groupe quinolone de formule (A'), dans lequel R'₆ est de l'hydrogène, au moins deux éléments parmi R'₂, R'₃, R'₄ et R'₅ sont des groupes -O-R₈ et R₈ est un groupe alkyle de C1-C20.

11. Procédé de photopolymérisation de compositions et d'encres photopolymérisables, comprenant les étapes suivantes consistant à :
(i) préparer ou fournir une composition photopolymérisable comprenant :
- des composés (a) et (b), tels que définis dans la revendication 1 ou
- des composés (a), (b) tels que définis dans la revendication 6, et un ou plusieurs composants choisis parmi les composants (c), (d), (e) et (f) tels que définis dans la revendication 6, de préférence (d) ;
(ii) photopolymériser la composition de l'étape (i) à l'aide d'une source de lumière.

12. Procédé de la revendication 11 est **caractérisé en ce que** ladite source de lumière comprend de la lumière UV dans au moins une des gammes UVA, UVB et UVC.

13. Procédé de la revendication 11 est **caractérisé en ce que** la source de lumière est une source LED émettant dans la gamme de 350 à 420 nm.

14. Procédé de l'une quelconque des revendications 11 à 13, comprenant en outre l'étape consistant à appliquer ladite composition photopolymérisable sur un substrat avant de le photopolymériser.

15. Article manufacturé obtenu selon le procédé de l'une quelconque des revendications 11 à 14 ou par impression tridimensionnelle d'un mélange comprenant la composition selon l'une quelconque des revendications 1 à 6.
